# EUROPEAN PATENT SPECIFICATION

(11) **EP 0 846 683 B1**
(45) Date of publication and mention of the grant of the patent: **19.09.2001**
(21) Application number: 97121040.6
(22) Date of filing: 01.12.1997
(51) Int. Cl.: C07D 211/48, C07D 405/06, A61K 31/445, A61K 31/34, A61K 31/35, C07D 401/06

(54) **4-Hydroxy-piperidine derivatives**
4-Hydroxypiperidinderivate
Dérivés de la 4-hydroxypipéridine

(30) Priority: 03.12.1996 EP 96119345
(43) Date of publication of application: 10.06.1998
(73) Proprietor: F. HOFFMANN-LA ROCHE AG, 4070 Basel (CH)
(72) Inventor: Alanine, Alexander, 68400 Riedisheim (FR); Büttelmann, Bernd, 79650 Schopfheim (DE); Heitz Neidhart, Marie-Paule, 68220 Hagenthal-le-Bas (FR); Pinard, Emmanuel, 68480 Linsdorf (FR); Wyler, René, 8002 Zürich (CH)
(74) Representative: Poppe, Regina

(56) References cited:
- EP-A- 0 546 389
- WO-A-91/17156
- DE-A- 1 695 836
- DE-A- 2 507 782
- DE-A- 2 627 616
- DE-A- 3 620 354
- DE-A- 3 620 408
- US-A- 5 436 255

## Description

The present invention relates to 4-hydroxy-piperidine derivatives of the general formula 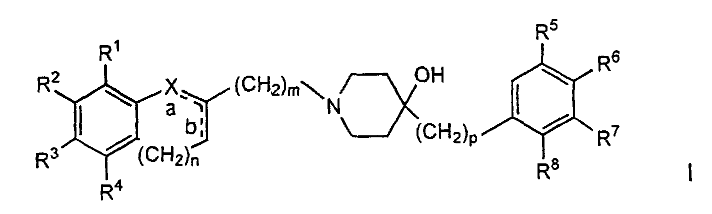 wherein
- X: denotes -O-, -NH-, -CH₂-, -CH=, -CHOH-, -S-, -SO- or -SO₂-;
- R¹-R⁴: are, independently from each other, hydrogen, hydroxy, C₁-C₄-alkyl-sulfonylamino, 1- or 2-imidazolyl or acetamido;
- R⁵-R⁸: are, independently from each other, hydrogen, hydroxy, C₁-C₄-alkyl, halogen, C₁-C₄-alkoxy, trifluoromethyl or trifluoromethyloxy;
- a and b: may be a double bond, provided that when "a" is a double bond, "b" cannot be a double bond;
- n: is 0-2;
- m: is 1-3;
- P: is 1
and to pharmaceutically acceptable addition salts thereof.

The compounds of formula I and their salts are distinguished by valuable therapeutic properties. Compounds of the present invention are NMDA(N-methyl-D-aspartate)-receptor subtype selective blockers, which have a key function in modulating neuronal activity and plasticity which makes them key players in mediating processes underlying development of the CNS including learning and memory formation and function.

Under pathological conditions of acute and chronic forms of neurodegeneration overactivation of NMDA receptors is a key event for triggering neuronal cell death. NMDA receptors are composed of members of two subunit families, namely NR-1 (8 different splice variants) and NR-2 (A to D) originating from different genes. Members from the two subunit families show a distinct distribution in different brain areas. Heteromeric combinations of NR-1 members with different NR-2 subunits result in NMDA receptors displaying different pharmaceutical properties. Possible therapeutic indications for NMDA receptor subtype specific blockers include acute forms of neurodegeneration caused, e.g., by stroke and brain trauma, and chronic forms of neurodegeneration such as Alzheimer's disease, Parkinson's disease, Huntington's disease, ALS (amyotrophic lateral sclerosis) and neurodegeneration associated with bacterial or viral infections.

Objects of the invention are the compounds of formula I and pharmaceutically acceptable addition salts thereof, racemic mixtures and their corresponding enantiomers, the preparation of the above-mentioned compounds, medicaments containing them and their manufacture as well as the use of the above-mentioned compounds in the control or prevention of illnesses, especially of illnesses and disorders of the kind referred to earlier, or for the manufacture of corresponding medicaments.

The following definitions of the general terms used in the present description apply irrespective of whether the terms in question appear alone or in combination.

As used herein, the term "lower alkyl" denotes a straight or branchedchain alkyl group containing from 1 to 4 carbon atoms, for example, methyl, ethyl, propyl, isopropyl, n-butyl, i-butyl, 2-butyl and t-butyl.

The term "halogen" denotes chlorine, iodine, fluorine and bromine.

The term "lower alkoxy" denotes a group wherein the alkyl residue is as defined above.

The term "leaving group" has the meaning conventionally used, and refers to, for example, halogen, alkylsulfonyloxy, arylsulfonyloxy and the like. The most preferred leaving group in the present case is a halogen.

The term "pharmaceutically acceptable addition salts" embraces salts with inorganic and organic acids generally known to a person skilled in the art, such as hydrochloric acid, nitric acid, sulfuric acid, phosphoric acid, citric acid, formic acid, fumaric acid, maleic acid, acetic acid, succinic acid, tartaric acid, methane-sulfonic acid, p-toluenesulfonic acid and the like.

Compounds of formula I wherein X is -O-, -NH-, -CHOH or -CH2- are preferred.

Exemplary preferred compounds in which X denotes -O-, are:
(RS)-1-(5-hydroxy-2,3-dihydro-benzofuran-2-ylmethyl)-4-(4-methyl-benzyl)-piperidine-4-ol,
(RS)-4-benzyl-1-(5-hydroxy-2,3-dihydro-benzofuran-2-ylmethyl)-piperidine-4-ol,
(RS)-4-(4-fluoro-benzyl)-1-(5-hydroxy-2,3-dihydro-benzofuran-2-ylmethyl)-piperidine-4-ol,
(RS)-4-(4-ethyl-benzyl)-1-(5-hydroxy-2,3-dihydro-benzofuran-2-ylmethyl)-piperidine-4-ol,
(S)-1-(5-hydroxy-2,3-dihydro-benzofuran-2-ylmethyl)-4-(4-methyl-benzyl)-piperidine-4-ol,
(S)-1-(5-hydroxy-2,3-dihydro-benzofuran-2-ylmethyl)-4-(4-chloro-benzyl)-piperidine-4-ol,
(RS)-N-[2-{4-hydroxy-4-(4-methyl-benzyl)-piperidine-1-ylmethyl)-2,3-dihydrobenzofuran-5-yl]-methane sulfonamide and
(RS)-N-[2-{4-hydroxy-4-(4-methyl-benzyl)-piperidine-1-ylmethyl}-2,3-dihydrobenzofuran-5-yl]-methane sulfonamide.

Exemplary preferred compounds in which X denotes -CHOH- are:
(1RS,2RS) and (1RS,2SR)-2-[4-hydroxy-4-(4-methyl-benzyl)-piperidine-1-ylmethyl]-indan-1,5-diol,
(1RS,2RS)-1-(1,6-dihydroxy-1,2,3,4-tetrahydro-naphthalen-2-ylmethyl)-4-(4-methyl-benzyl)-piperidine-4-ol and
(1RS,2RS)-2-(4-benzyl-4-hydroxy-piperidine-1-yl-methyl)-6-hydroxy-1,2,3,4-tetrahydronaphthalen-1-ol.

Other exemplary preferred compounds in which X denotes -CH₂- are:
(RS)-1-(5-hydroxy-indan-2-ylmethyl)-4-(4-methyl-benzyl)-piperidine-4-ol and
(RS)-1-(6-hydroxy-1,2,3,4-tetrahydro-naphthalen-2-ylmethyl) -4-(4-methyl-benzyl)-piperidine-4-ol.

Furthermore, another preferred compound in which X denotes -NH- is (RS)-2-[4-hydroxy-4-(4-methyl-benzyl)-piperidine-1-ylmethyl]-2,3-dihydro-1H-indol-5-ol.

The present compounds of formula I and their pharmaceutically acceptable salts can be prepared by methods known in the art, for example, by processes described below, which comprises:
a) reacting a compound of the formula 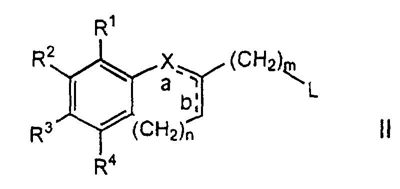
   wherein R¹-R⁴, X, a, b, n and m have the meaning given above and L is OH or a leaving group, for example, halogen or -O-tosyl,
      with a compound of the formula 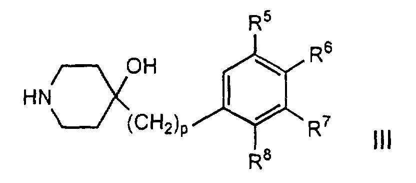
   wherein R⁵-R⁸ and p have the significances given above,
      or
b) dehydrating a compound of the formula 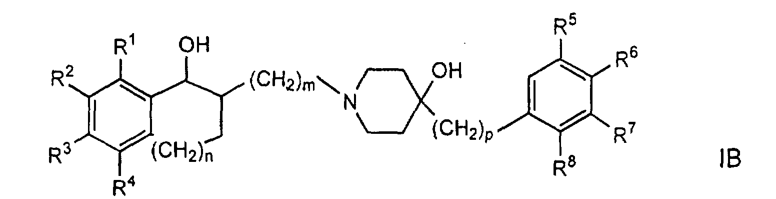 to give a compound of the formula 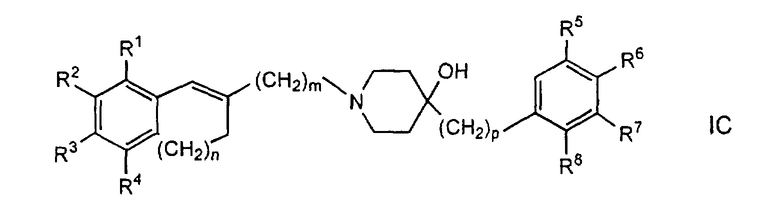 wherein m is 1 and the other substituents have the significances given above, or
c) reducing a compound of the formula 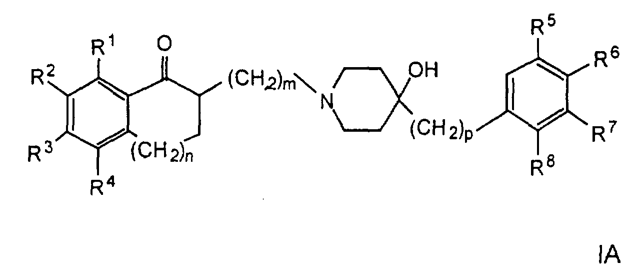 to give a compound of the formula IB, or
d) debenzylating a compound of the formula 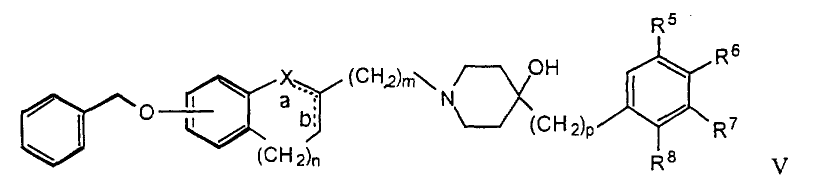
e) reacting a compound of formula I, wherein one of R¹-R⁴ is an amino group with a lower-alkyl-sulfonyl halogen to give a compound of formula I, wherein one of R¹-R⁴ is a lower-alkyl-sulfonyl-amino group, or
f) hydrogenating the isolated double bond in a compound of formula I, or
g) cleaving off (a) hydroxy or amino protecting group(s) present as (a) substituent(s) R¹-R⁴ or as X' = -N(protecting group)-, or
h) oxidizing a compound of formula I, wherein X represents -S- or -SO- to yield the corresponding sulfonyl (-SO₂) compound, and
i) if desired, converting the compound of formula I obtained into a pharmaceutically acceptable addition salt.

In accordance with process variant a) a mixture of a compound of formula II and of formula III, wherein the leaving group L in formula II is, for example, bromine, was dissolved in a suitable solvent, for example in DMF and heated to about 80-90°C. This reaction is carried out in the presence of a base, preferred is triethylamine. The compound of formula I is then separated in conventional manner. When one of R¹-R⁴ in formula II is a hydroxy group these groups are protected by groups conventionally used.

Examples of such groups are described in green, T. Protective Groups in Organic Synthesis, Chapter 7, John Wiley and Sons, Inc. (1981), pp 218-287. Most preferred are the benzyloxy or alkyloxy groups. This reaction can be carried out by known methods, for example by hydrogenation with Pd/C (10%) or borontribromide-dichloromethane solution.

In accordance with process variant b) a compound of formula IB can be dehydrated in the presence of ethanolic HCl in conventional manner. Obtained are compounds of formula I in which "a" represents a double bond.

Variant c) describes a method for reducing of compounds of formula IA to give compounds of the formula IB. This reaction is carried out in conventional manner, preferred is the presence of LiAlH₄ in THF and at temperature of about 5-10°C.

In accordance with process variant d) a compound of formula I is obtained, wherein one of R¹-R⁴ is hydroxy. This process is carried out by debenzylating a compound of formula V, provided that none of R⁵-R⁸ is halogen. The debenzylation is carried out in conventional manner. For example, a compound of formula V is dissolved in a suitable solvent or mixture of solvents such as ethanol and ethylacetate, and hydrogenated in the presence of Pd on C at room temperature and atmospheric pressure.

In accordance with process variant e) a compound of formula I can be obtained, wherein one of R¹-R⁴ is a lower-alkyl-sulfonyl-amino group. This reaction is carried out by treating a compound of formula I, wherein one of R¹-R⁴ is an amino group, with a lower-alkyl-sulfonylhalogen, such as methane sulfonylchloride, in a suitable solvent, such as methylene chloride, in the presence of pyridine at room temperature.

The hydrogenation of a compound of formula I, wherein one of "a" or "b" is a double bond in accordance with process variant f) is carried out in conventional manner, for example in the presence of Pd/C in ethylacetate under hydrogen atmospher for about 24 hours at room temperature. Protecting groups, for example the hydroxy group, can be cleaved off by methods described above. Suitable protecting groups and methods for their cleavage will be familiar to any person skilled in the art; although of course there can be used only those protecting groups which can be cleaved off by
methods under conditions of which other structural elements in the compounds are not affected.

The oxidation of compounds of formula I, wherein X is -S- or -SO-, is carried out in conventional manner. In accordance with process variant g) a compound of formula I, wherein X represents -S- or -SO-, is oxidized to yield the corresponding sulfonyl (SO₂-) compound. The oxidation can be carried out in the presence of Oxone® (potassium monopersulfate triple salt) at room temperature or in the presence of metachloroperbenzoic acid.

The addition salts of the compounds of formula I are especially well suited for pharmaceutical use.

The starting materials for the preparation of compounds of formula I are known or can be prepared by known methods, for example, according to the following reaction schemes. These reactions are described in more detail in Examples 33-75. 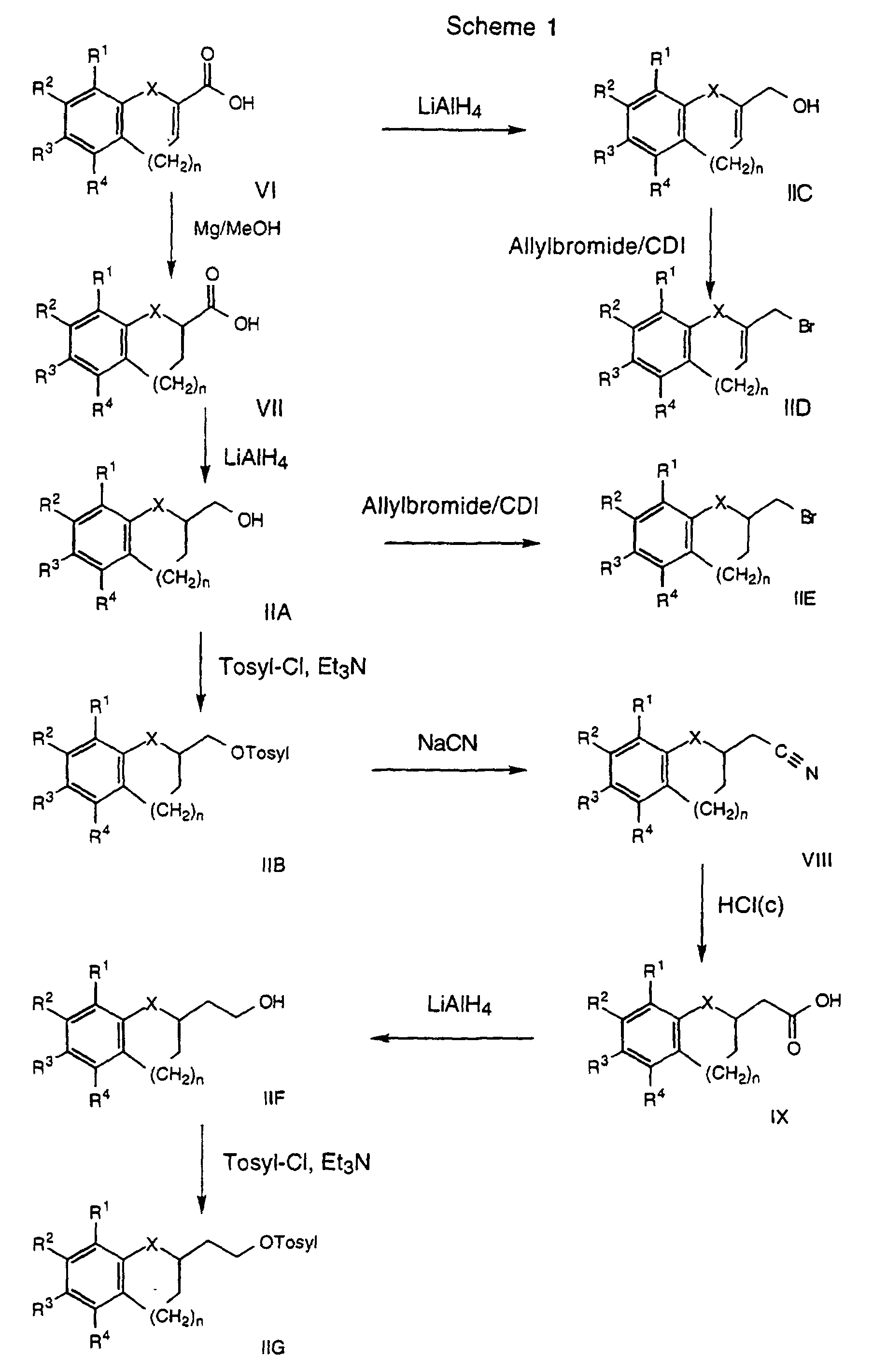 wherein the meaning of the substituents is as given above. 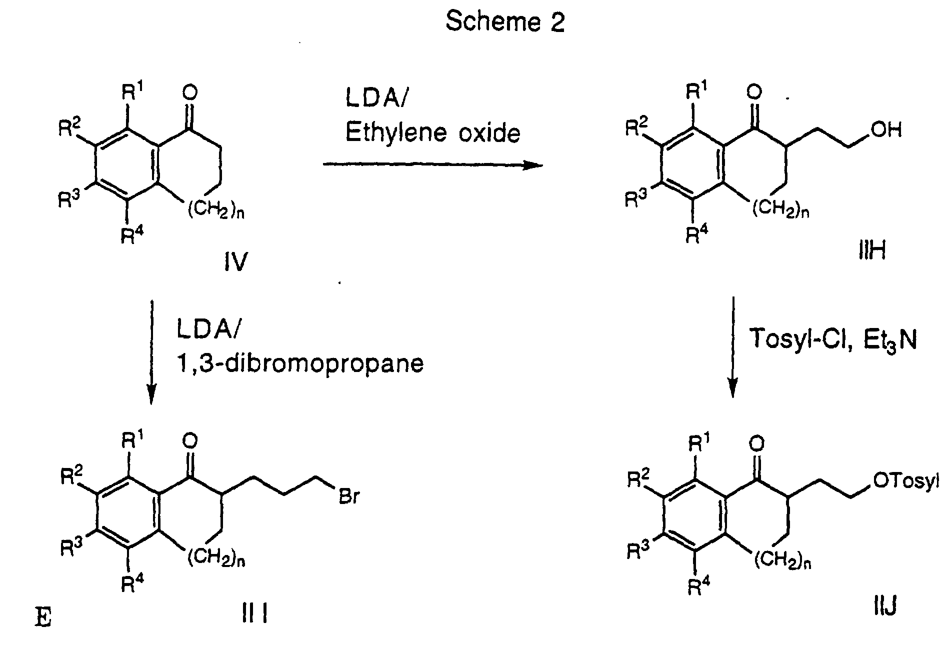 wherein the meaning of the substituents is as given above. 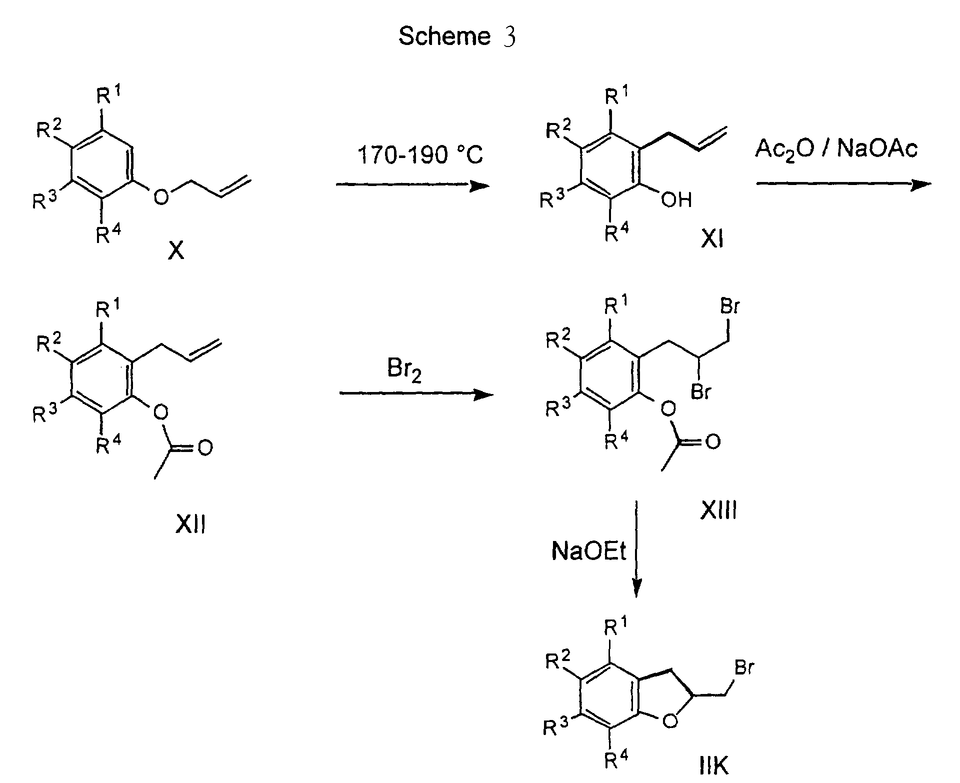 wherein the meaning of the substituents is as given above. 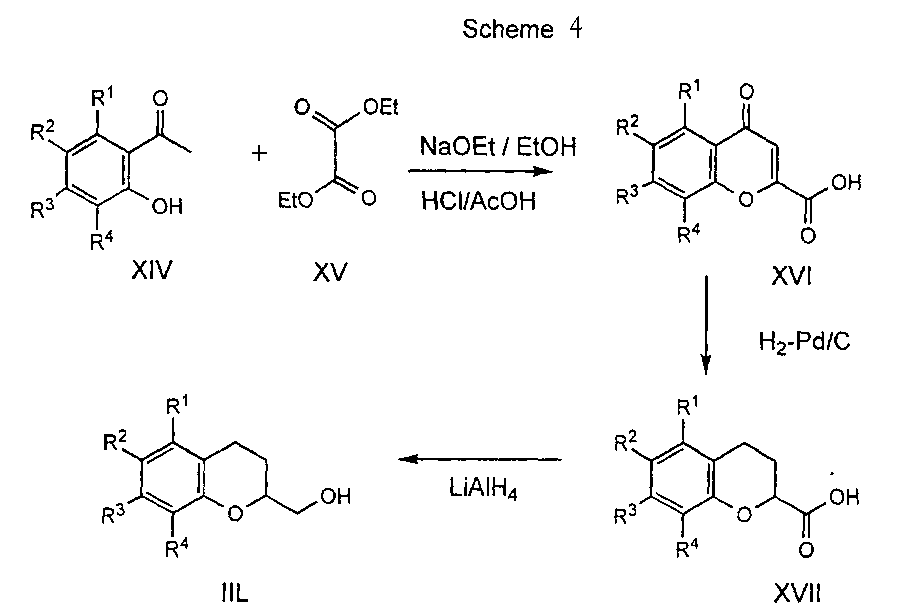 wherein the meaning of the substituents is as given above. 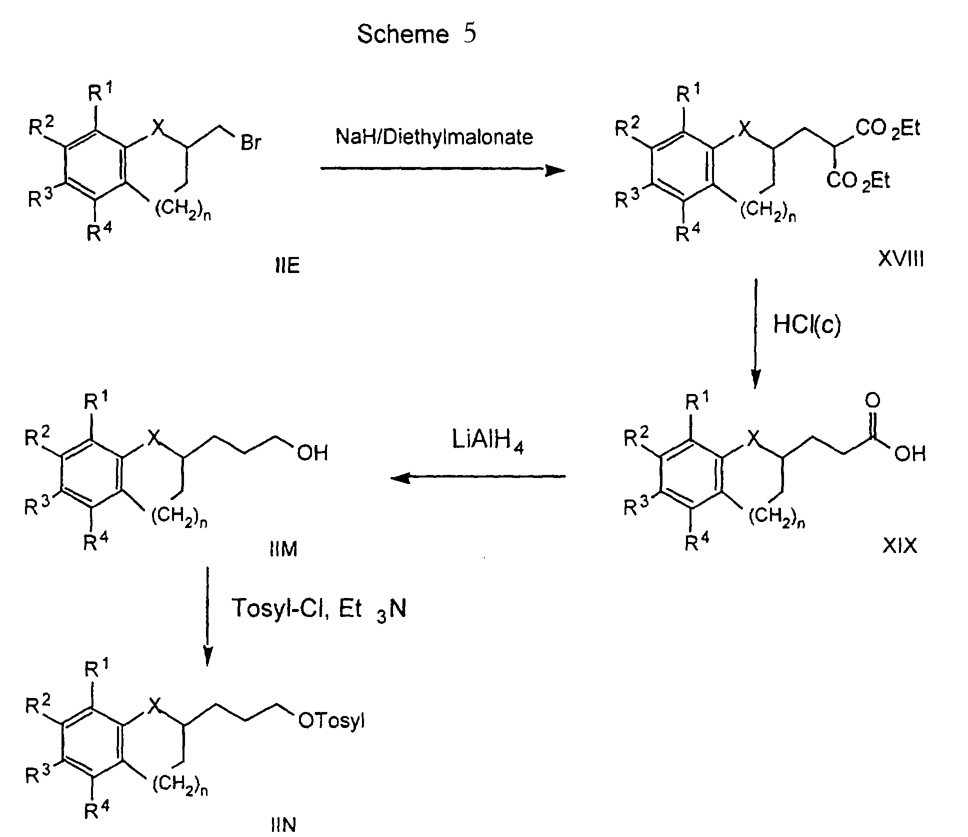 wherein the meaning of the substituents is as given above.

As mentioned earlier, the compounds of formula I and their pharmaceutically usable addition salts possess valuable pharmacodynamic properties. They are NMDA-receptor subtype selective blockers, which have a key function in modulating neuronal activity and plasticity which makes them key players in mediating processes underlying development of CNS as well as learning and memory formation.

The compounds were investigated in accordance with the tests given hereinafter.

### Method 1

### 3H-Ro 25-6981 binding (Ro 25-6981 is [R-(R*,S*)]-a-(4-Hydroxy-phenyl)-b-methyl-4-(phenyl-methyl)-1-piperidine propanol)

Male Füllinsdorf albino rats weighing between 150-200 g were used. Membranes were prepared by homogenization of the whole brain minus cerebellum and medulla oblongata with a Polytron (10.000 rpm, 30 seconds), in 25 volumes of a cold Tris-HCl 50 mM, EDTA 10 mM, pH 7.1 buffer. The homogenate was centrifuged at 48.000 g for 10 minutes at 4°C. The pellet was resuspended using the Polytron in the same volume of buffer and the homogenate was incubated at 37°C for 10 minutes. After centrifugation the pellet was homogenized in the same buffer and frozen at -80°C for at least 16 hours but not more than 10 days. For the binding assay the homogenate was thawed at 37°C, centrifuged and the pellet was washed three times as above in a Tris-HCl 5 mM, pH 7.4 cold buffer. The final pellet was resuspended in the same buffer and used at a final concentration of 200 µg of protein/ml.

3H-Ro 25-6981 binding experiments were performed using a Tris-HCl 50 mM, pH 7.4 buffer. For displacement experiments 5 nM of 3H-Ro 25-6981 were used and non specific binding was measured using 10 µM of tetrahydroisoquinoline and usually it accounts for 10% of the total. The incubation time was 2 hours at 4°C and the assay was stopped by filtration on Whatmann GF/B glass fiber filters (Unifilter-96, Packard, Zürich, Switzerland). The filters were washed 5 times with cold buffer. The radioactivity on the filter was counted on a Packard Top-count microplate scintillation counter after addition of 40 mL of microscint 40 (Canberra Packard S.A., Zürich, Switzerland).

The effects of compounds were measured using a minimum of 8 concentrations and repeated at least once. The pooled normalized values were analyzed using a non-linear regression calculation program which provide IC₅₀ with their relative upper and lower 95% confidence limits (RS1, BBN, USA).

### Method 2

### 3H-Prazosine binding

Male Füllinsdorf albino rats weighing between 150-200 g were used. Membranes were prepared by homogenization of the whole brain minus cerebellum and medulla oblongata with a Plytron (10.000 rpm, 30 seconds), in 25 volumes of a cold Tris-HCl 50 mM, EDTA 10mM, pH 7.1 buffer. The homogenate was centrifuged at 48.000 g for 10 minutes at 4°C. The pellet was resuspended using the Polytron in the same volume of buffer and the homogenate was incubated at 37°C for 10 minutes. After centrifugation the pellet was homogenized in the same buffer and frozen at -80°C for at least 16 hours but not more than 10 days. For the binding assay the homogenate was thawed at 37°C, centrifuged and the pellet was washed three times as above in a Tris-HCl 5mM, pH 7.4 cold buffer. The final pellet was resuspended in the same buffer and used at a final concentration of 200 µg of protein/ml.

3H-Prazosine binding experiments were performed using a Tris-HCl 50 mM, pH 7.4 buffer. For displacement experiments 0.2 nM of 3H-Prazosine were used and non specific binding was measured using 100 µM of Chlorpromazine. The incubation time was 30 minutes at room temperature and the assay was stopped by filtration on Whatman GF/B glass fiber filters (Unifilter-96, Canberra Packard S.A., Zürich, Switzerland). The filters were washed 5 times with cold buffer. The radioactivity on the filter was counted on a Packard Top-count microplate scintillation counter after addition of 40 ml of microscint 40 (Canberra Packard S.A., Zürich, Switzerland). The effects of compounds were measured using a minimum of 8 concentrations and repeated at least once. The pooled normalized values were analyzed using a non-linear regression calculation program which provide IC₅₀ with their relative upper and lower 95% confidence limits (RS1, BBN, USA).

### Method 3

### Electrophysiology on recombinant NMDA receptors

cDNA clones coding for the subunits NR1C and NR2A of the NMDA receptor (see Hollmann and Heinemann, 1994, Annu. Rev. Neurosci. 17:31 for nomenclature of NMDA receptor subunits) were isolated from a rat brain λgt11 cDNA library as published elsewhere (Sigel et al., 1994, J. Biol. Chem. 269:8204). The clone for the subunit NR2B of the rat brain NMDA receptor was obtained from S. Nakanishi (Kyoto, Japan). The cDNAs encoding rat NR1C, NR2A and NR2B were subcloned into the expression vector pBC/CMV (Bertocci et al., 1991, Proc. Natl. Acad. Sci. U.S.A. 88:1416), placing transcription of the cDNA under control of the human cytomegalovirus promoter. CsCl-purified expression plasmids were mixed in a 1:3 ratio of NR1C:NR2A or NR1C:NR2B in injection buffer (88 mM NaCI, 1 mM KCl, 15 mM HEPES, at pH 7.0). Oocytes of South African frogs (Xenopus laevis) were used for expressing either a combination of the NR1C and NR2A subunits or the NR1C and NR2B subunits. 12 to 120 pg of a 1:3 (NR1C:NR2B) mixture of the respective cDNA species were injected into the nucleus of every oocyte. On the following two days the ion current through the NMDA receptor channels was measured in voltage clamp experiments for the methods of cDNA expression in oocytes and voltage-clamping (see, e.g., Bertrand et al., 1991, Methods in Neurosciences 4:174). The membrane potential was clamped to -80 mV and the receptors were activated by applying a modified Ringer's solution containing the NMDA-receptor agonists L-glutamate (Glu) and glycine (Gly). Different agonist concentrations were chosen for either subunit combination to account for the different agonist sensitivities of the two types of receptors (2.7 µM Glu plus 0.9 µM Gly for NR1C + NR2A and 1.3 µM Glu plus 0.07 µM Gly for NR1C + NR2B). The agonists were applied for 15 s intervals once every 2.5 min by rapid superfusion of the oocyte with agonist containing solution and the amplitude of the agonist-evoked current was measured immediately before the end of each application. After a series of initial control applications the antagonist to be tested was added to both, the basal Ringer's and the agonist containing solution. The antagonist concentration applied to oocytes expressing the NR2A subunit was 10 µmol/l, whereas 0.1 µmol/l were applied to the NR2B expressing oocytes. Four to six oocytes were tested for every compound and NMDA receptor subtype. Oocytes were exposed to the compounds for 5 to 30 min depending on the time needed for reaching an equilibrium block of the NMDA receptor current. For every oocyte the decrease of the current amplitude was expressed as a percentage of the control current measured before application of the compound. Figures in the table are arithmetic mean values of these percentage values.

The thus-determined activity of some compounds in accordance with the invention will be evident from the following table.

| Compound/ Example | 3H-Ro-25-6981 binding IC₅₀ (µM) | 3H-prazosine Binding IC₅₀ (µM) | Electrophysiology NR1C + NR2A NR1C + NR2B % block by 10 µM 0.1 µM | |
|---|---|---|---|---|
| 1 | 0.040 | 3.0 | 65* | 72 |
| 2 | 0.020 | 3.2 | | |
| 4 | 0.050 | 2.5 | | |
| 5 | 0.040 | 4.0 | | |
| 7 | 0.012 | 6.0 | 12 | 89 |
| 13 | 0.014 | 6.0 | 8 | 93 |
| 14 | 0.005 | 6.0 | 9 | 90 |
| 16 | 0.023 | 5.0 | 6 | 86 |
| 19 | 0.055 | 3.2 | | |
| 21 | 0.055 | 6.6 | | |
| 22 | 0.040 | 6.5 | | |
| 23 | 0.040 | 2.3 | | |
| 27 | 0.026 | 6.0 | 15 | 91 |
| 29 | 0.023 | 11.0 | | |
| 30 | 0.020 | 0.8 | | |

| | | | | |
|---|---|---|---|---|
| * 33% block at a concentration of 1 µM | | | | |

**(01)** 1-(6-Hydroxy-3,4-dihydro-naphthalene-2-ylmethyl)-4-(4-methyl-benzyl)-piperidine-4-ol
**(02)** (RS)-1-(5-Hydroxy-2,3-dihydro-benzofuran-2-ylmethyl)-4-(4-methyl-benzyl)-piperidin-4-ol hydrochloride
**(04)** (RS)-4-Benzyl-1-(5-hydroxy-2,3-dihydro-benzofuran-2-ylmethyl)-piperidin-4-ol hydrochloide
**(05)** (RS)-4-(4-fluoro-benzyl)-1-(5-hydroxy-2,3-dihydro-benzofuran-2-ylmethyl)-piperidin-4-ol hydrochloride
**(07)** (RS)-4-(4-ethyl-benzyl)-1-(5-hydroxy-2,3-dihydro-benzofuran-2-ylmethyl)-piperidin-4-ol hydrochloride
**(13)** (S)-1-(5-Hydroxy-2,3-dihydro-benzofuran-2-ylmethyl)-4-(4-methyl-benzyl)-piperidin-4-ol hydrochloride
**(14)** (S)-1-(5-Hydroxy-2,3-dihydro-benzofuran-2-ylmethyl)-4-(4-chloro-benzyl)-piperidin-4-ol hydrochloride
**(16)** (1RS,2RS) and (1RS, 2SR)-2-[4-Hydroxy-4-(4-methyl-benzyl)-piperidine-1-ylmethyl]-indan-1,5-diol fumarate salt
**(19)** (RS)-1-(5-Hydroxy-indan-2-ylmethyl)-4-(4-methyl-benzyl)-piperidin-4-ol
**(21)** (RS)-2-[4-Hydroxy-4-(4-methyl-benzyl)-piperidin-1-ylmethyl]-2,3-dihydro-1H-indol-5-ol
**(22)** (RS)-N(2-[4-Hydroxy-4-{4-methyl-benzyl}-piperidin-1-ylmethyl]-2,3-dihydrobenzofuran-5-yl)-methane sulfonamide hydrochloride
**(23)** (RS)-N(2-[4-Hydroxy-4-{4-methyl-benzyl}-piperidin-1-ylmethyl]-2,3-dihydrobenzofuran-5-yl)-methane sulfonamide hydrochloride
**(27)** (1RS, 2RS)-1-(1,6-Dihydroxy-1,2,3,4-tetrahydro-naphthalen-2-ylmethyl)-4-(4-methyl-benzyl)-piperidin-4-ol fumarate salt
**(29)** (1RS,2RS)-2-(4-benzyl-4-hydroxy-piperidin-1-yl-methyl)-6-hydroxy-1,2,3,4-tetrahydro-naphthalen-1-ol
**(30)** (RS)-1-(6-Hydroxy-1,2,3,4-tetrahydro-naphthalen-2-ylmethyl)-4-(4-methyl-benzyl)-piperidin-4-ol hydrochloride

By screening compounds of formula I could be identified as NMDA receptor subtype selective blockers and - for selected compounds - the preference for NMDAR-2B subunits could be demonstrated by eletrophysiological characterization using cloned NMDA receptor subtypes expressed oocytes.

The compounds of formula I and their salts, as herein described, can be incorporated into standard pharmaceutical dosage forms, for example, for oral or parenteral application with the usual pharmaceutical adjuvant materials, for example, organic or inorganic inert carrier materials, such as, water, gelatin, lactose, starch, magnesium stearate, talc, vegetable oils, gums, polyalkylene-glycols and the like. The pharmaceutical preparations can be employed in a solid form, for example, as tablets, suppositories, capsules, or in liquid form, for example, as solutions, suspensions or emulsions. Pharmaceutical adjuvant materials can be added and include preservatives stabilizers, wetting or emulsifying agents, salts to change the osmotic pressure or to act as buffers. The pharmaceutical preparations can also contain other therapeutically active substances.

The daily dose of compounds of formula I to be administered varies with the particular compound employed, the chosen route of administration and the recipient. Representative of a method for administering the compounds of formula I is by the oral and parenteral type administration route. An oral formulation of a compound of formula I is preferably administered to an adult at a dose in the range of 150 mg to 1.5 g per day. A parenteral formulation of a compound of formula I is preferably administered to an adult at a dose in the range of 5 to 500 mg per day.

The following Examples illustrate the invention in more detail. All temperatures are given in degrees Celsius.

### Example 1

### 1 -(6-Hydroxy-3.4-dihydro-naphthalene-2-ylmethyl)-4-(4-methyl-benzyl)-piperidine-4-ol

(1RS, 2RS) (1-1,6-dihydroxy-1,2,3,4-tetrahydro-naphthalen-2-ylmethyl)-4-(4-methyl-benzyl)-piperidin-4-ol (286 mg, 0.75 mmol) was dissolved in EtOAc (25 ml) and treated with 6.4 N HCl in EtOH at RT. The mixture was then heated to reflux for 1 hr. After cooling, H₂O was added (25 ml) and the mixture neutralised with 10% NaHCO₃ solution. The reaction mixture was then extracted with EtOAc (2x 50 ml), washed with satd. NaCl solution (25 ml), dried with Na₂SO₄, filtered and evaporated to afford the title compound as a pink solid (238.8 mg, 0.657 mmol, 87 %); MS: m/e= 363.2 (M+H⁺).

### Example 2

### (RS)-1-(5-Hydroxy-2,3-dihydro-benzofuran-2-ylmethyl)-4-(4-methyl-benzyl)-piperidin-4-ol hydrochloride

(RS)-1-(5-benzyloxy-2,3-dihydro-benzofuran-2-ylmethyl)-4-(4-methyl-benzyl)-piperidin-4-ol (1.0 g , 2.2 mmol) in MeOH (100 ml) was hydrogenated with Pd/C (10%) (200 mg) for 17 hr at ambient temperature. Removal of the catalyst and evaporation afforded a yellow foam (720 mg, 2.0 mmol, 92 %). This material (618 mg, 1.75 mmol) was then dissolved in EtOH (20 ml) and treated with 1.45N HCl/EtOH (1.1 eq.) at 0-5 °C to afford (RS)-1-(5-hydroxy-2,3-dihydro-benzofuran-2-ylmethyl)-4-(4-methyl-benzyl)-piperidin-4-ol hydrochloride as a white/beige foam, E/Z isomer mixture (679 mg, quant.), MS: m/e= 354.2 (M+H⁺).

Following the method of example 2 the compounds of examples 3 to 12 were prepared

### Example 3

### (RS)-1-(5-Hydroxy-2,3-dihydro-benzofuran-2-ylmethyl)-4-(4-methoxy-benzyl)-piperidin-4-ol hydrochloride

The title compound MS: m/e= 370.2 (M+H⁺) was prepared from (RS)-1-(5-benzyloxy-2,3-dihydro-benzofuran-2-ylmethyl)-4-(4-methoxy-benzyl)-piperidin-4-ol.

### Example 4

### (RS)-4-Benzyl-1-(5-hydroxy-2,3-dihydro-benzofuran-2-ylmethyl)-piperidin-4-ol hydrochloride

The title compound MS: m/e= 340.2 (M+H⁺) was prepared from (RS)-4-benzyl-1-(5-benzyloxy-2,3-dihydro-benzofuran-2-ylmethyl)-piperidin-4-ol.

### Example 5

### (RS)-4-(4-Fluoro-benzyl)-1-(5-hvdroxv-2.3-dihvdro-benzofuran-2-ylmethyl)-piperidin-4-ol hydrochloride.

The title compound MS: m/e= 358.2 (M+H⁺) was prepared from (RS)-4-(4-fluoro-benzyl)-1-(5-hydroxy-2,3-dihydro-benzofuran-2-ylmethyl)-piperidin-4-ol.

### Example 6

### (RS)-4-(3,4-Dimethyl-benzyl)-1-(5-hydroxy-2,3-dihydro-benzofuran-2-ylmethyl)-piperidin-4-ol hydrochloride

The title compound MS: m/e= 368.2 (M+H⁺) was prepared from (RS)-4-(3,4-dimethyl-benzyl)-1-(5-benzyloxy-2,3-dihydro-benzofuran-2-ylmethyl)-piperidin-4-ol.

### Example 7

### (RS)-4-(4-Ethyl-benzyl)-1-(5-hydroxy-2,3-dihydro-benzofuran-2-ylmethyl)-piperidin-4-ol hydrochloride

The title compound MS: m/e= 368.2 (M+H⁺) was prepared from (RS)-4-(4-ethyl-benzyl)-1-(5-benzyloxy-2,3-dihydro-benzofuran-2-ylmethyl)-piperidin-4-ol.

### Example 8

### (RS)-4-(4-Isopropyl-benzyl)-1-(5-hydroxy-2,3-dihydro-benzofuran-2-ylmethyl)-piperidin-4-ol hydrochloride

The title compound MS: m/e= 382.2 (M+H⁺) was prepared from (RS)-4-(4-isopropyl-benzyl)-1-(5-benzyloxy-2,3-dihydro-benzofuran-2-ylmethyl)-piperidin-4-ol.

### Example 9

### (RS)-4-(2-Methyl-benzyl)-1-(5-hydroxy-2,3-dihydro-benzofuran-2-ylmethyl)-piperidin-4-ol hydrochloride

The title compound MS: m/e=354.2 (M+H⁺) was prepared from (RS)-4-(2-methyl-benzyl)-1-(5-benzyloxy-2,3-dihydro-benzofuran-2-ylmethyl)-piperidin-4-ol.

### Example 10

### (RS)-4-(2,4-Difluoro-benzyl)-1-(5-hydroxy-2,3-dihvdro-benzofuran-2-ylmethyl)-piperidin-4-ol hydrochloride.

The title compound MS: m/e= 376.2 (M+H⁺) was prepared from (RS)-4-(2,4-difluoro-benzyl)-1-(5-benzloxy-2,3-dihydro-benzofuran-2-ylmethyl)-piperidin-4-ol.

### Example 11

### (RS)-4-(4-Trifluoromethoxy-benzyl)-1-(5-hydroxy-2,3-dihydro-benzofuran-2-ylmethyl)-piperidin-4-ol hydrochloride.

The title compound MS: m/e= 424.2 (M+H⁺) was prepared from (RS)-4-(4-trifluoro-methoxy-benzyl)-1-(5-benzyloxy-2,3-dihydro-benzofuran-2-ylmethyl)-piperidin-4-ol.

### Example 12

### (RS)-4-(3-Trifluoromethyl-benzyl)-1-(5-hydroxy-2,3-dihydro-benzofuran-2-ylmethyl)-piperidin-4-ol hydrochloride.

The title compound MS: m/e= 408.2 (M+H⁺) was prepared from (RS)-4-(3-trifluoro-methyl-benzyl)-1-(5-benzyloxy-2,3-dihydro-benzofuran-2-ylmethyl)-piperidin-4-ol.

### Example 13

### (S)-1-(5-Hvdroxy-2,3-dihydro-benzofuran-2-vlmethyl)-4-(4-methyl-benzyl)-piperidin-4-ol hydrochloride

(S)-1-(5-methoxy-2,3-dihydro-benzofuran-2-ylmethyl)-4-(4-methyl-benzyl)-piperidin-4-ol (2.10g, 5.71 mmol) dissolved in 50 ml of CH₂Cl₂ was cooled to -78 °C and 1M BBr₃-CH₂Cl₂ solution (12.5 ml, 2.2 eq.) was added dropwise under argon. The beige suspension was allowed to warm to ambient temperature over 30 min and then stirred for a further 1 hour, during which time a sticky yellow solid deposited. MeOH (10 ml) was then added to quench the reaction, followed by distilled H₂O (100 ml) and satd. NaHCO₃ solution (25 ml); the mixture was then stirred vigorously for 15 min. The organic phase was separated, satd. NaCl solution (100 ml) was then added to the aqueous phase and extracted with CH₂Cl₂ (2x 50 ml). The combined organic extracts were dried with Na₂SO₄ then filtered and evaporated. The resulting yellow foam was chromatographed over SiO₂ (Merck 230-400 mesh) eluting with CH₂Cl₂ followed by MeOH-CH₂Cl₂ (3:97) followed by MeOH-CH₂Cl₂ (7:93) (1.7 g, 4.81 mmol, 84% yield). (S)-1-(5-hydroxy-2,3-dihydro-benzofuran-2-ylmethyl)-4-(methyl-benzyl)-piperidin-4-ol (1.62 g, 4.58 mmol) was suspended in EtOH and treated with 1.1 eq of ethanolic HCl at 0-5°C affording (S)-1-(5-hydroxy-2,3-dihydro-benzofuran-2-ylmethyl)-4-(4-methyl-benzyl)-piperidin-4-ol hydrochloride as a white foam and mixture of E/Z isomers (1.74 g, 4.46 mmol, 97%) MS: m/e= 354.2 (M+H⁺) [α]²⁰_{D}= +57.8° (c=1.0, EtOH)

Following the general method of example 13, compound of examples 14 and 15 were prepared.

### Example 14

### (S)-1-(5-Hydroxy-2,3-dihydro-benzofuran-2-ylmethyl)-4-(4-chloro-benzyl)-piperidin-4-ol hydrochloride

The title compound, MS: m/e= 374.2 (M+H⁺) [α]²⁰_{D} = +32.4° (c=1.0, DMF), >99% e.e. by chiral phase HPLC, was prepared from (S)-1-(5-methoxy-2,3-dihydro-benzofuran-2-ylmethyl)-4-(4-chloro-benzyl)-piperidin-4-ol.

### Example 15

### (R)-1-(5-Hydroxy-2,3-dihydro-benzofuran-2-ylmethyl)-4-(methyl-benzyl)-piperidin-4-ol hydrochloride

The title compound as a white foam and mixture of E/Z isomers (1.64 g, 4.20 mmol, 100 %) MS: m/e= 354.2 (M+H⁺) [α]²⁰_{D} = -58.0° (c=1.0, EtOH) was prepared from (R)-1-(5-methoxy-2,3-dihydro-benzofuran-2-ylmethyl)-4-(methyl-benzyl)-piperidin-4-ol.

### Example 16

### (1RS,2RS) and (1RS,2SR)-2-[4-Hydroxy-4-(4-methyl-benzyl)-piperidine-1-ylmethyl]-indan-1,5-diol fumarate salt

(1RS,2RS) and (1RS, 2SR)-2-[4-benzyloxy-4-(4-methyl-benzyl)-piperidine-1-ylmethyl]-indan-1,5-diol (674 mg, 1.84 mmol) was taken up in EtOH (20 ml), fumaric acid (106 mg, 0.92 mmol) was added and the mixture stirred for 2 hr at RT, after evaporation of the solvent, (1RS,2RS) and (1RS, 2SR)-2-[4-hydroxy-4-(4-methyl-benzyl)-piperidine-1-ylmethyl]-indan-1,5-diol fumaric acid salt was obtained as a white foam (0.78 g, quant.), MS m/e= 368.2 (M+H⁺).

### Example 17

### (2RS,2RS) and (1RS, 2SR)-2-[4-hydroxy-4-(4-methyl-benzyl)-piperidine-1-ylmethyl]-indan-1,5-diol

A solution of (1RS,2RS)and (1RS,2SR)-2-[4-benzyloxy-4-(4-methyl-benzyl)-piperidine-1-ylmethyl]-indan-1,5-diol (0.83 g, 1.82 mmol) in MeOH (100 ml) and Pd/C 10% (100 mg) was stirred vigorously under a hydrogen atmosphere for 1 hr at ambient temperature. After removal of the catalyst and evaporation of the solvent the title compound (1RS,2RS) and (1RS, 2SR)-2-[4-hydroxy-4-(4-methyl-benzyl)-piperidine-1-ylmethyl]-indan-1,5-diol (664 mg, 1.81 mmol, 99%) was afforded as a white amorphous foam, (1:1) mixture of diastereoisomers, MS m/e= 368.2 (M+H⁺).

### Example 18

### (RS)-1-(5-Hydroxy-indan-2-ylmethyl)-4-(4-methyl-benzyl)-piperidin-4-ol hydrochloride

(RS)-1-(5-hydroxy-indan-2-ylmethyl)-4-(4-methyl-benzyl)-piperidin-4-ol (177 mg, 0.503 mmol) was dissolved in EtOH and ethanolic HCl (1.7 eq) was added, the product was precipitated after 15 min. by addition of diethylether while cooling to 4 °C. The product was afforded as a white solid-foam (RS)-1-(5-hydroxy-indan-2-ylmethyl)-4-(4-methyl-benzyl)-piperidin-4-ol hydrochloride (95.7 mg, 0.246 mmol, 49%) Mp. 88-90 °C, MS m/e= 352.2 (M+H⁺).

### Example 19

### (RS)-1-(5-Hydroxy-indan-2-vlmethyl)-4-(4-methyl-benzyl)-piperidin-4-ol

1-(6-Hydroxy-1H-inden-2-ylmethyl)-4-(4-methyl-benzyl)-piperidin-4-ol (256 mg, 0.732 mmol) and Pd/C 10% (50 mg) in EtOAc (15 ml) was stirred vigorously under a hydrogen atmosphere for 24 hr at RT. Removal of the catalyst and evaporation of the solvent afforded (RS)-1-(5-hydroxy-indan-2-ylmethyl)-4-(4-methyl-benzyl)-piperidin-4-ol as a colourless oil (235 mg, 0.667 mmol, 91%), MS m/e= 352.2 (M+H⁺).

### Example 20

### 1-(6-Hydroxy-1H-inden-2-ylmethyl)-4-(4-methyl-benzyl)-piperidin-4-ol.

(1RS,2RS) and (1RS, 2SR)-2-[4-hydroxy-4-(4-methyl-benzyl)-piperidine-1-ylmethyl]-indan-1,5-diol (310 mg, 0.84 mmol) and ethanolic HCl (5 eq.) was heated in EtOAc (30 ml) at 65 °C for 1.5 hr. Distilled H₂O (30 ml) and 10% NaHCO₃ (30 ml) was added and the mixture shaken, the aqueous phase was further extracted with EtOAc (2x 20 ml) and the combined organic extracts were washed with satd. NaCl solution (30 ml), dried (Na₂SO₄) and filtered. Evaporation of the solvent afforded 1-(6-hydroxy-1H-inden-2-ylmethyl)-4-(4-methyl-benzyl)-piperidin-4-ol as a beige solid (308 mg, 0.84 mmol, 100 %) Mp. 154-157 °C, MS m/e= 350.2 (M+H⁺).

### Example 21

### (RS)-2-[4-Hydroxy-4-(4-methyl-benzyl)-piperidin-1-ylmethyl]-2,3-dihydro-1H-indol-5-ol

To (RS)-1-(5-methoxy-2,3-dihydro-1H-indol-2-ylmethyl)-4-(4-methyl-benzyl)-piperidin-4-ol (131 mg, 0.357 mol) in CH₂Cl₂ (10 ml) was added 1M BBr₃-CH₂Cl₂ (2.14 ml, 2.74 mmol, 6 eq.) at -78 °C over 5 min. The reaction was stirred at RT for 48 hr., then MeOH (20 ml) was added followed by 10% NaHCO₃ (20 ml), and the aqueous phase extracted with CH₂Cl₂ (2x 50 ml) and the combined extracts washed with satd. NaCl solution (50 ml), dried over (Na₂SO₄) filtered and evaporated. Purification of the crude product over SiO₂ (Merck 230-400 mesh) eluting with CH₂Cl₂-MeOH (9:1) afforded (RS)-2-[4-hydroxy-4-(4-methyl-benzyl)-piperidin-1-ylmethyl]-2,3-dihydro-lH-indol-5-ol as a brown solid (64.6 mg, 0.183 mmol, 51%), Mp. 90-94 °C, MS: m/e= 353.3 (M+H⁺).

### Example 22

### (RS)-N-{2-[4-(4-chloro-benzyl)-4-hydroxy-pipiridin-1-ylmethyl]-2,3-dihydroxybenzofuran-5-yl}methane sulfonamide hydrochloride.

(RS)-N-(2-bromomethyl-2,3-dihydro-benzofuran-5-yl)-methansulfonamide (600 mg, 1.96 mmol), 4-(4-chloro-benzyl)-piperidin-4-ol (514 mg, 1.96 mmol), Et₃N(400 mg, 3.96 mmol) were dissolved in DMF (50ml) and heated at 60 °C for 90 hrs. DMF was then evapored, the residue was dissolved in CH₂Cl₂ and washed with H₂O. The organic phase was dried over Na₂SO₄, and concentrated. The residue was chromatographed over SiO₂ (Merck 230-400 mesh) eluting with CH₂Cl₂-MeOH-NH₄OH (65:10:1) to provide a beige foam which was dissolved in THF (50ml) and treated with 1.2N HCl (lml) to afford (RS)-N-{2-[4-(4-chloro-benzyl)-4-hydroxy-pipiridin-1-ylmethyl]-2,3-dihydroxybenzofuran-5-yl}methane sulfonamide hydrochloride as a white foam and mixture of E/Z isomers. MS: m/e= 451.3 (M+H⁺)

Following the method of example 22 the compound of example 23 was prepared.

### Example 23

### (RS)-N(2-[4-hydroxy-4-(4-methyl-benzyl)-piperidin-1-ylmethyl]-2,3-dihydrobenzofuran-5-yl]-methane sulfonamide hydrochloride.

The title compound MS: m/e=431.5 (M+H⁺) was prepared from (RS)-N-(2-bromomethyl-2,3-dihydro-benzofuran-5-yl)-methansulfonamide and 4-(4-Methyl-benzyl)-piperidin-4-ol.

### Example 24

### (RS)-1-(6-Hydroxy-2,3-dihydro-benzofuran-2-ylmethyl)-4-(4-methyl-benzyl)-piperidin-4-ol hydrochloride

(RS)-1-(6-methoxy-2,3-dihydro-benzofuran-2-ylmethyl)-4-(4-methyl-benzyl)-piperidin-4-ol (1.24g, 3.37 mmol) dissolved in 35 ml of CH₂Cl₂ was cooled to -8 °C and 1M BBr₃-CH₂Cl₂ solution (6.8 ml, 2 eq.) was added dropwise under argon. The violet suspension was allowed to warm to room temperature and then stirred for 30 minutes. The reaction was then cooled to 0 °C and MeOH (9 ml) was added to quench the reaction, followed by satd. NaHCO₃ (50 ml). The organic phase was separated and the aqueous phase was extracted with CH₂Cl₂ (2x 50 ml). The combined organic extracts were dried with Na₂SO₄ then filtered and evaporated. The resulting yellow foam was chromatographed over SiO₂ (Merck 230-400 mesh) eluting with MeOH-CH₂Cl₂ (1:19) followed by MeOH-CH₂Cl₂ (1:9) to provide a yellow foam which was dissolved in MeOH (5 ml) and treated with 1N HCl (3.4 ml) to provide (RS)-1-(6-hydroxy-2,3-dihydrobenzofuran-2-ylmethyl)-4-(methyl-benzyl)-piperidin-4-ol hydrochloride (0.92g, 70%) as a white solid and mixture of E/Z isomers. Mp. 203-205°C MS: m/e= 354.3(M+H⁺)

Following the method of example 24 the compound of example 25 was prepared.

### Example 25

### 1-(6-Hydroxy-benzofuran-2-yl-methyl)-4-(4-methyl-benzyl)-piperidin-4-ol hydrochloride

The title compound m.p. 214 °C and MS: m/e=352.2 (M+H⁺) was prepared from 1-(6-methoxy-benzofuran-2-ylmethyl)-4-(4-methyl-benzyl)-piperidin-4-ol.

### Example 26

### (RS)-4-Benzyl-1-(6-hydroxy-chroman-2-ylmethyl)-piperidin-4-ol hydrochloride

(RS)-4-Benzyl-1-(6-benzyloxy-chroman-2-ylmethyl)-piperidin-4-ol (0.58 g, 1.31 mmol) was dissolved in EtOAc (30 ml) and Pd/C 10% (135 mg) was added, the mixture was placed under an atmosphere of hydrogen and stirred vigorously for 17 hr at ambient temperature. Removal of the catalyst and chromatography over SiO₂ (Merck 230-400 mesh) CH₂Cl₂-MeOH-NH₄OH (100:5:0.25) afforded a white foam (0.37 g, 1.04 mmol, 80 %) which was taken up in EtOH (10 ml) and HCl/EtOH (1.1 eq.) was added at 0-5°C. Removal of the solvent gave (RS)-4-benzyl-1-(6-hydroxy-chroman-2-ylmethyl)-piperidin-4-ol hydrochloride (0.38 g, 0.98 mmol, 95%) a white foam, MS: m/e= 354.4 (M+H⁺).

### Example 27

### (1RS,2RS) (1-(1,6-Dihydroxy-1,2,3,4-tetrahydro-naphthalen-2-ylmethyl)-4-(4-methyl-benzyl)-piperidin-4-ol fumarate salt

(1RS, 2RS) (1-(1,6-dihydroxy-1,2,3,4-tetrahydro-naphthalen-2-ylmethyl)-4-(4-methyl-benzyl)-piperidin-4-ol (0.581g, 1.53 mmol) in EtOH (20 ml) was stirred with fumaric acid (88 mg, 0.765 mmol, 0.5 eq) for 2 hr at RT. The mixture was then completely evaporated and dried under high vacuum to afford the title compound as an amorphous white foam (0.66g, quant.), MS:m/e= 382.3 (M+H⁺).

### Example 28

### (1RS, 2RS) (1-(1,6-Dihydroxy-1,2,3,4-tetrahydro-naphthalen-2-ylmethyl)-4-(4-methyl-benzyl)-piperidin-4-ol

1-(6-Benzyloxy-1-hydroxy-1,2,3,4-tetrahydro-naphthalen-2-ylmethyl)-4-(4-methyl-benzyl)-piperidin-4-ol (0.22 g, 0.46 mmol) in EtOAc (60 ml) was treated with Pd/C 10% (50 mg) and stirred for 6 hr at ambient temperature under an atmosphere of hydrogen. Removal of the catalyst and evaporation of the solvent afforded the title compound as a white foam (175 mg, quant.), MS m/e= 382.3 (M+H⁺).

### Example 29

Following the general method of example 28 compound of example 29 was prepared.

### (1RS,2RS)-2-(4-Benzyl-4-hydroxy-piperidin-1-yl-methyl)-6-hydroxy-1,2,3,4-tetrahydro-naphthalen-1-ol.

The title compound was obtained as a white solid Mp. 94-98 °C, MS: m/e= 368.4 (M+H⁺), prepared from (1RS,2RS)-2-(4-benzyl-4-hydroxy-piperidin-1-yl-methyl)-6-benzyloxy-1,2,3,4-tetrahydro-naphthalen-1-ol.

### Example 30

### (RS)-1-(6-Hydroxy-1,2,3,4-tetrahvdro-naphthalen-2-ylmethyl)-4-(4-methyl-benzyl)-piperidin-4-ol hydrochloride.

To (RS)-1-(6-hydroxy-1,2,3,4-tetrahydro-naphthalen-2-ylmethyl)-4-(4-methyl-benzyl)-piperidin-4-ol (500 mg, 1.36 mmol) in EtOH (4 ml) at 4 °C, was added ethanolic HCl (1.1 eq.). Evaporation of the EtOH afforded the title compound as a white foam (530 mg, 1.32 mmol, 97 %), MS m/e= 366.2 (M+H⁺).

### Example 31

### (RS)- 1-(6-Hydroxy-1,2,3,4-tetrahydro-naphthalen-2-ylmethyl)-4-(4-methyl-benzyl)-piperidin-4-ol

To (RS)-1-(6-methoxy-1,2,3,4-tetrahydro-naphthalen-2-ylmethyl)-4-(4-methyl-benzyl)-piperidin-4-ol (843 mg, 2.22 mmol) in CH₂Cl₂ (20 ml) was added 1M BBr₃/CH₂Cl₂ (4.88 ml, 4.88 mmol, 2.2 eq.) over 15 min at -78 °C, the mixture was then allowed to warm to RT over 40 min. MeOH (3 ml), H₂O (20 ml) and NaHCO₃ (20 ml) were added and the mixture extracted with CH₂Cl₂ (4x50 ml). The extracts were washed with satd. NaCl solution (30 ml), dried (Na₂SO₄), filtered and evaporated, to afford the crude product as a yellow foam. Chromatography over SiO₂ (Merck 230-400 mesh) eluting with CH₂Cl₂-MeOH (97:3) afforded (RS)-1-(6-hydroxy-1,2,3,4-tetrahydro-naphthalen-2-ylmethyl)-4-(4-methyl-benzyl)-piperidin-4-ol as a white foam (500 mg, 13.68 mmol, 61%), MS m/e= 366.2 (M+H⁺).

### Example 32

### (RS)-N-[6-(4-Benzyl-4-hydroxy-piperidin-1-ylmethyl)-5-oxo-5,6,7,8-tetrahydro-naphthalen-2-yl]-acetamide hydrochloride

N-(5,6,7,8-tetrahydro-5-oxo-2-naphthyl)acetamide (1.0g, 4.92 mmol), 4-(benzyl)-piperidin-4-ol hydrochloride (1.12 g, 4.92 mmol) and paraformaldehyde (148 mg, 4.92 mmol) were heated together in DMF (50 ml) at 80 °C for 4 hr. The DMF was then evaporated and the residue taken up in CH₂Cl₂ (50 ml) and washed with 10% NaHCO₃ (25 ml), the aqueous phase was further extracted with CH₂Cl₂(50 ml) and the combined CH₂Cl₂ extracts were dried (Na₂SO₄) filtered and evaporated. The crude material was chromatographed over SiO₂ (Merck 230-400 mesh) eluting with CH₂Cl₂-MeOH-NH₄OH (110:10:1) affording 0.79 g of a yellow foam. This material was dissolved in EtOH cooled to 0-4 °C and ethanolic HCl (1.1 eq.) added, the white solid which precipitated was collected and dried to afford (RS)-N-[6-(4-benzyl-4-hydroxy-piperidin-1-ylmethyl)-5-oxo-5,6,7,8-tetrahydro-naphthalen-2-yl]-acetamide hydrochloride (639 mg, 1.44 mmol, 29%), Mp. 123-126 °C, MS: m/e= 407.5 (M+H⁺).

N-(5,6,7,8-Tetrahydro-2-naphthyl)acetamide was prepared according to the literature:
Biggs,D.F. et al., *J*.*Med*.*Chem*., 19, **1976,** 472-475.; Allinger,N.L.; Jones,E.S., *J.Org.Chem.,* 27, **1962,** 70-76.

### Preparation of intermediates

### General preparation of benzyl piperidin intermediates

### Example 33

### (RS)-1-(5-Benzyloxy-2,3-dihydro-benzofuran-2-ylmethyl)-4-(4-methyl-benzyl)-piperidin-4-ol

(RS)-5-Benzyloxy-2-bromomethyl-2,3-dihydro-benzofuran (840 mg, 2.63 mmol) and 4-(4-methyl-benzyl)-piperidin-4-ol (1.08 g, 5.26 mmol) were suspended in toluene (20 ml) and heated to 110 °C for 17 hr. The mixture was filtered and evaporated to afford an orange oil which was chromatographed over SiO₂ (Merck 230-400 mesh) with MeOH-CH₂Cl₂ (3:97) to afford (RS)-1-(5-benzyloxy-2,3-dihydro-benzofuran-2-ylmethyl)-4-(4-methyl-benzyl)-piperidin-4-ol as a yellow oil (1.03 g, 2.32 mmol, 88%), MS: m/e= 444.5 (M+H⁺).

### Preparation of intermediates for examples 2-12

Following the general method of example 33, examples 34 to 43 were prepared.

### Example 34

### 1-(5-Benzyloxy-2,3-dihydro-benzofuran-2-ylmethyl)-4-(4-methoxy-benzyl)-piperidin-4-ol.

The title compound MS: m/e= 460.2 (M+H⁺) was prepared from 4-(4-methoxy-benzyl)-piperidin-4-ol and 5-benzyloxy-2-(RS)-bromomethyl-2,3-dihydro-benzofuran.

### Example 35

### (RS)-4-Benzyl-1-(5-benzyloxy-2,3-dihydro-benzofuran-2-ylmethyl)-piperidin-4-ol

The title compound MS: m/e= 430.6 (M+H⁺) was prepared from 4-(benzyl)-piperidin-4-ol and 5-benzyloxy-2-(RS)-bromomethyl-2,3-dihydro-benzofuran.

### Example 36

### (RS)-4-(4-Fluoro-benzyl)- 1-(5-benzyloxy-2,3-dihydro-benzofuran-2-ylmethyl)-piperidin-4-ol.

The title compound MS: m/e= 448.6 (M+H⁺) was prepared from 4-(4-fluoro-benzyl)-piperidin-4-ol and 5-benzyloxy-2-(RS)-bromomethyl-2,3-dihydro-benzofuran.

### Example 37

### (RS)-4-(3,4-Dimethyl-benzyl)-1-(5-benzyloxy-2,3-dihydro-benzofuran-2-ylmethvl)-piperidin-4-ol

The title compound MS: m/e= 458.6 (M+H⁺) was prepared from 4-(3,4-dimethyl-benzyl)-piperidin-4-ol and 5-benzyloxy-2-(RS)-bromomethyl-2,3-dihydro-benzofuran.

### Example 38

### (RS)-4-(4-Ethyl-benzyl)-1-(5-benzyloxy-2,3-dihydro-benzofuran-2-ylmethyl)-piperidin-4-ol.

The title compound MS: m/e= 458.6 (M+H⁺) was prepared from 4-(4-ethyl-benzyl)-piperidin-4-ol and 5-benzyloxy-2-(RS)-bromomethyl-2,3-dihydro-benzofuran.

### Example 39

### (RS)-4-(4-Isopropyl-benzyl)-1-(5-benzyloxy-2,3-dihydro-benzofuran-2-ylmethyl)-piperidin-4-ol

The title compound MS: m/e= 472.6 (M+H⁺) was prepared from 4-(4-isopropyl-benzyl)-piperidin-4-ol and 5-benzyloxy-2-(RS)-bromomethyl-2,3-dihydro-benzofuran.

### Example 40

### (RS)-4-(2-Methyl-benzyl)-1-(5-benzyloxy-2,3-dihydro-benzofuran-2-ylmethyl)-piperidin-4-ol

The title compound MS: m/e=444.6 (M+H⁺) was prepared from 4-(2-methyl-benzyl)-piperidin-4-ol and 5-benzyloxy-2-(RS)-bromomethyl-2,3-dihydro-benzofuran.

### Example 41

### (RS)-4-(2,4-Difluoro-benzyl)-1-(5-benzyloxy-2,3-dihydro-benzofuran-2-vlmethvl)-piperidin-4-ol.

The title compound MS: m/e= 466.6 (M+H⁺) was prepared from 4-(2,4-difluoro-benzyl)-piperidin-4-ol and 5-benzyloxy-2-(RS)-bromomethyl-2,3-dihydro-benzofuran.

### Example 42

### (RS)-4-(4-Trifluoromethoxy-benzyl)-1-(5-benzyloxy-2,3-dihydro-benzofuran-2-ylmethyl)-piperidin-4-ol.

The title compound MS: m/e= 514.6 (M+H⁺) was prepared from 4-(4-trifluoromethoxy-benzyl)-piperidin-4-ol and 5-benzyloxy-2-(RS)-bromomethyl-2,3-dihydro-benzofuran.

### Example 43

### (RS)-4-(3-Trifluoromethyl-benzyl)-1-(5-benzyloxy-2,3-dihydro-benzofuran-2-ylmethyl)-piperidin-4-ol.

The title compound MS: m/e= 498.6 (M+H⁺) was prepared from 4-(3-trifluoromethyl-benzyl)-piperidin-4-ol and 5-benzyloxy-2-(RS)-bromomethyl-2,3-dihydro-benzofuran.

### Example 44

### (RS)-5-Benzyloxy-2-(bromomethyl-2,3-dihydro-benzofuran

(RS)-Acetic acid 4-benzyloxy-2-(2,3-dibromo-propyl)-phenyl ester (5.05 g, 11.3 mmol) was suspended in EtOH (50 ml) and sodium methoxide (620 mg, 11.3 mmol) added and the mixture stirred for 2 hr at ambient temperature. Distilled H₂O (100 ml) and CH₂Cl₂ (100 ml) was then added and the organic phase separated. The aqueous phase was extracted with CH₂Cl₂ (100 ml) and the combined organic extracts washed with satd. NaCl solution (100 ml). After drying with Na₂SO₄, filtration and evaporation a yellow oil resulted which was chomatographed over SiO₂ (Merck 230-400 mesh) with CH₂Cl₂ to afford 5-benzyloxy-2-(RS)-bromomethyl-2,3-dihydro-benzofuran as a yellow oil (3.0 g, 9.39 mmol, 83%), MS: m/e= 318.0 (M⁺).

### Example 45

### (RS)-Acetic acid 4-benzyloxy-2-(2,3-dibromo-propyl)-phenyl ester

To a solution of acetic acid-2-allyl-4-benzyloxy-phenyl ester (3.30 g, 11.7 mmol) in CCl₄ (30 ml) at 0-5 °C, bromine (0.6 ml, 11.7 mmol) was added over 10 min. and the resulting mixture stirred for 1 hr at 5-10 °C. Na₂CO₃ solution (10%, 4 ml) and distilled H20 (10 ml) were added to quench the reaction. The organic phase was separated, dried with Na₂SO₄ and then evaporated to afford a colourless oil, which crystallised to provide on standing (RS)-acetic acid 4-benzyloxy-2-(2,3-dibromo-propyl)-phenyl ester (5.05 g, 11.4 mmol, 97%), Mp. 72-74 °C, MS: m/e= 440.0 (M⁺).

### Example 46

### Acetic acid-2-allyl-4-benzyloxy-phenyl ester

2-Allyl-4-benzyloxy-phenol (2.87 g, 12 mmol) was taken up in acetic anhydride (40 ml) and NaOAc (150 mg, 1.8 mmol) was added and the mixture heated 18 hr at 80 °C. After cooling, the reaction mixture was evaporated to afford an oil which was partitioned between EtOAc and H20 (100 ml) and the aqueous phase was extracted with EtOAc (100 ml) and the combined organic phases were dried with MgSO₄, filtered and evaporated to afford acetic acid-2-allyl-4-benzyloxy-phenyl ester as a pale yellow oil (3.30 g, 11.7 mmol, 97%), MS: m/e= 282.1 (M⁺).

### Example 47

### 2-Allyl-4-benzyloxy-phenol

1-Benzyloxy-4-allyloxy-benzene (20.4 g, 84.9 mmol) dissolved in mesitylene (150 ml) was heated at 165 °C for 48 hr under an argon atmosphere. After cooling to ambient temperature the resulting brown oil was chromatographed over SiO₂ (Merck 230-400 mesh) eluting with EtOAc-nHexane (1:9) to afford 2-allyl-4-benzyloxy-phenol (17.45 g, 72.6 mmol, 85%) as a pale yellow oil MS m/e= 240.1 (M⁺).

### Example 48

### 1-Benzyloxy-4-allyloxy-benzene

4-Benzyloxyphenol (20 g, 100 mol), K₂CO₃ (20.8 g, 150 mmol) and allyl bromide (12.7 ml, 150 mmol) were heated under reflux in acetone (200 ml) 18 hr. After filtration and evaporation of the solvent 1-benzyloxy-4-allyloxy-benzene (23.8 g, 99 mmol, 99 %) was afforded as a beige solid Mp. 56-57 °C, MS m/e= 240.1 (M⁺).

### Preparation of the intermediate for examples 13 and 14.

### Example 49

### (S)-1-(5-Methoxy-2,3-dihydro-benzofuran-2-ylmethyl)-4-(4-methyl-benzyl)-piperidin-4-ol

(S)-Toluenesulfonic acid 5-methoxy-2,3-dihydro-benzofuran-2-ylmethyl ester (2.30g, 6.88 mmol) and 4-(4-methyl-benzyl)-piperidin-4-ol (1.62 g, 7.9 mmol) and Na₂CO₃ (1.10 g, 10.3 mmol) were suspended in DMF and heated at 110 °C for 1 hr. After cooling to ambient temperature distilled H₂O and EtOAc were added (100 ml) and the mixture shaken, the organic phase was then separated and the aqueous phase extracted with EtOAc (10 ml). The combined organic extracts were then washed with satd. NaCl solution (100 ml) and the organic phase dried with Na₂SO₄, filtered and evaporated to afford a yellow oil. This foam was chromatographed over SiO₂ (Merck 230-400 mesh) with CH₂Cl₂, and then with CH₂Cl₂-MeOH (97:3) to afford (S)-1-(5-methoxy-2,3-dihydro-benzofuran-2-ylmethyl)-4-(4-methyl-benzyl)-piperidin-4-ol (2.20 g, 6.0 mmol, 87% yield) as a yellow oil MS: m/e= 368.2 (M+H⁺) [α]²⁰_{D} = +45.8° (c=1.0, CHCl₃).

Following the general method of example 49, example 50 was prepared.

### Example 50

### (S)-1-(5-Methoxy-2.3-dihydro-benzofuran-2-ylmethyl)-4-(4-chloro-benzyl)-piperidin-4-ol

The title compound, MS: m/e= 387.9 (M+H⁺), was prepared from 4-(4-chloro-benzyl)-piperidin-4-ol and (S)-toluenesulfonic acid 5-methoxy-2,3-dihydro-benzofuran-2-ylmethyl ester.

### Example 51

### (S)-Toluenesulfonic acid 5-methoxy-2,3-dihydro-benzofuran-2-ylmethyl ester

A solution of (S)-5-methoxy-2,3-dihydro-benzofuran-2-carboxylic acid (2.4g, 12.4 mmol) in THF (30 ml) was added dropwise to a suspension of LiAlH₄ (0.71 g, 18.5 mmol) in THF (20 ml) over 15 min with cooling. The mixture was then heated to reflux for 1 hr., after cooling distilled H20 (0.7 ml) was added followed by 4N NaOH (1.4 ml) and distilled H₂O (2.1 ml). The whole mixture was dried with Na₂SO₄, filtered and evaporated to afford a light yellow oil (2.20 g, 12.2 mmol, 100 %). This oil was dissolved in pyridine (22 ml) and *p*-toluene sulfonyl chloride was added (3.48 g, 18.3 mmol), and the mixture stirred at ambient temperature for 1 hr. H₂O was then added to the crude mixture and vigorously stirrred for 10 min., extraction with EtOAc (2x100ml) and washing the organic phase with 2N HCl (150 ml) followed by satd. NaCl (100 ml), drying with Na₂SO₄ and evaporation afforded a yellow oil. This oil was chromatographed over SiO₂ (Merck 230-400 mesh) with cyclohexane-EtOAc (4:1) to afford (S)-toluenesulfonic acid 5-methoxy-2,3-dihydro-benzofuran-2-ylmethyl ester (2.4g 7.2 mmol, 57%) as a white solid Mp. 82-84 °C, MS: m/e= 334.1 (M⁺) [α]²⁰_{D} = +75.1° (c=1.0, CHCl₃).

### Example 52

### (S)-5-Methoxy-2,3-dihydro-benzofuran-2-carboxylic acid

To a solution of R(+)-1-(1-naphthyl)ethylamin (3.80 g, 22.2 mmol) in acetone (40 ml) was added (RS)-5-methoxy-2,3-dihydro-benzofuran-2-carboxylic acid (4.1 g, 21.1 mmol) in acetone (80 ml) after 2-3 min. stirring at ambient temperature a beige solid precipitated, the mixture was cooled to 0-5 °C and stirred for a further 30 min. The solid was filtered and washed with cold (4 °C) acetone (3x20 ml) to afford white crystals 6 g, Mp. 183-190 °C. This solid was recrystallised twice from hot EtOH (60 ml) to afford 4.0 g of white crystals Mp. 190-194 °C. This material was suspended in EtOAc (100 ml) and washed with 1N HCl (50 ml), the acidic aqueous phase was extracted with EtOAc (50 ml), the combined organic extracts were then washed with distilled H20 (50 ml) and then dried with Na₂SO₄ and evaporated to afford (S)-5-methoxy-2,3-dihydro-benzofuran-2-carboxylic acid (2.3 g, 11.8 mmol, 77 %) as a pale yellow crystalline solid Mp. 85-87 °C, MS: m/e= 194.2 (M⁺) [α]²⁰_{D} = -9.2° (c=1.0, EtOH).

### Example 53

### (RS)-5-Methoxy-2,3-dihydro-benzofuran-2-carboxvlic acid

To a suspension of 5-methoxy-benzofuran-2-carboxylic acid (14 g, 72.8 mmol) in MeOH (600 ml) was added magnesium turnings (10.6 g, 437 mmol) and the mixture was vigorously mechanically stirred for 2 hr keeping the temperature below 30 °C. After 2 hr, further magnesium was added (10.6 g, 437 mmol) and the mixture stirred a further 4 hr again maintaining the temperature below 30 °C. After 6 hr. the mixture was concentrated to -100 ml and distilled H₂O (600 ml) was added and the pH adjusted to 1-2 with 1N sulfuric acid. The crude mixture was extracted with EtOAc (2x 300 ml) and the combined extracts washed with H20 (200 ml). The combined organic phase was dried with Na₂SO₄, filtered and evaporated to afford yellowish solid, which was recrystallised from hot toluene (100 ml) to afford white crystals (9.2 g, 47.4 mmol, 65%) Mp. 98-100 °C, MS: m/e= 194.1 (M⁺).

Following the general method of example 49 compound of example 54 was prepared.

### Example 54

### (R)-1-(5-Methoxy-2,3-dihydro-benzofuran-2-ylmethyl)-4-(4-methyl-benzyl)-piperidin-4-ol

The title compound (2.40 g, 6.53 mmol, 76% yield) as a yellow oil MS: m/e= 368.2 (M+H⁺) [α]²⁰_{D}= -44.0° (c=1.0, CHCl₃) was prepared from (R)-toluenesulfonic acid 5-methoxy-2,3-dihydro-benzofuran-2-ylmethyl ester.

Following the general method of example 51, compound of example 55 was prepared.

### Example 55

### (R)-Toluenesulfonic acid 5-methoxy-2,3-dihydro-benzofuran-2-ylmethyl ester

The title compound (3.0 g 9.0 mmol, 67%) as a white solid Mp. 82-84 °C, MS: m/e= 334.1 (M+) [α]²⁰_{D} = -74.9° (c=1.0, CHCl₃) >99% e.e. by chiral phase HPLC was prepared from (R)-5-methoxy-2,3-dihydro-benzofuran-2-carboxylic acid.

### Example 56

### (R)-5-Methoxy-2,3-dihydro-benzofuran-2(R)-carboxylic acid

To a solution of S(-)-1-(1-naphthyl)ethylamin (7.21g, 41.6 mmol) in acetone (80 ml) was added (RS)-5-methoxy-2,3-dihydro-benzofuran-2-carboxylic acid (7.70 g, 39.6 mmol) in acetone (150 ml) after 2-3 min. stirring at ambient temperature a beige solid precipitated, the mixture was cooled to 0-5 °C and stirred for a further 30 min. The solid was filtered and washed with cold (4 °C) acetone (3x20 ml) to afford white crystals 9.90 g, Mp.145-160°C. This solid was recrystallised twice from hot EtOH (125 ml) to afford 4.75 g of white crystals Mp. 185-194 °C. This material was suspended in EtOAc (100 ml) and washed with 1N HCl (50 ml), the acidic aqueous phase was extracted with EtOAc (50 ml), the combined organic extracts were then washed with distilled H20 (50 ml) and then dried with Na₂SO₄ and evaporated to afford (R)-5-methoxy-2,3-dihydro-benzofuran-2-carboxylic acid (2.5 g, 12.9 mmol, 65 %) as a pale yellow crystalline solid Mp. 85-87 °C, MS: m/e= 194.1 (M⁺) [α]²⁰_{D} = +9.8° (c=1.0, EtOH).

### Example 57

### Mixture of (1RS,2RS) and (1RS, 2SR)-2-[4-benzyloxy-4-(4-methyl-benzyl)-piperidine-1-ylmethyl]-indan-1,5-diol

5-Benzyloxy-indan-1-one (2.38 g, 10 mmol), 4-(4-methyl-benzyl)-piperidin-4-ol hydrochloride (2.41 g, 10 mmol) and paraformaldehyde (0.3 g, 10 mmol) in DMF (20 ml) were heated at 70 °C for 20 hr. After cooling EtOAc (150 ml), distilled H₂O (200 ml) and 25% NH₄OH (4 ml) was added and the mixture shaken. The aqueous phase was further extracted with EtOAc (150 ml) and the combined organic extracts were washed with satd. NaCl (2x100 ml) and dried with Na₂SO₄, affording a yellow oil after evaporation. This oil was dissolved in THF (40 ml) and added over 30 min to a suspension of LiAlH₄ (1.87 g, 50 mmol, 5 eq.) in THF (50 ml) with ice cooling (5-15 °C). The mixture was then allowed to stir for a further 2 hr at RT, the reaction was quenched by the addition of distilled H₂O (2 ml), 4N NaOH (4 ml) then H₂O (4 ml) and stirred for 15 min vigorously. The whole mixture was then dried with Na₂SO₄, filtered, washed with THF and evaporated to afford a viscose oil. The crude product was chromatographed over SiO₂ (Merck 230-400 mesh) eluting with CH₂Cl₂-MeOH (97:3) then CH₂Cl₂-MeOH (9:1) to afford a white foam which could be crystallised from EtOAc-Et₂O affording (1RS,2RS) and (1RS, 2SR)-2-[4-benzyloxy-4-(4-methyl-benzyl)-piperidine-1-ylmethyl]-indan-1,5-diol as white crystals (1.89 g, 4.13 mmol, 41 %), Mp. 131-132 °C, MS m/e= 458.4 (M+H⁺).

### Example 58

### 5-Benzyloxy-indan-1-one

A mixture of 5-hydroxy-indan-1-one (5.3 g, 35.7 mmol), KJ (0.6 g, 3.6 mmol) and K₂CO₃ (6.17 g, 44.6 mmol) and benzyl bromide (4.66 ml, 39.3 mmol) in DMF (50 ml) were heated at 100 °C for 1 hr. Addition of H₂O (150 ml) and extraction with EtOAc (3x 50 ml), washing the organic phase with satd. NaCl solution (100 ml) and drying with MgSO₄ and evaporation afforded a brown solid. This material was recrystallised twice from EtOH to afford 5-benzyloxy-indan-1-one (6g, 25.17 mmol, 70%) as yellow crystals, Mp. 105-106 °C, MS m/e= 238.1 (M⁺).

### Example 59

### 5-Hydroxy-indan-1-one

5-Methoxy-indan-1-one (8 g, 49.3 mmol), and 4-tert-butyl-2-methyl-benzenethiol sodium salt (11.92 g, 59.2 mmol) were heated at 142 °C for 1 hr under argon. After cooling to RT, water (80 ml) was added followed by 1N HCl (80 ml) and EtOAc (120 ml). The mixture was shaken and the aqueous phase extracted with EtOAc (100 ml), the combined organic extracts were washed with satd.NaCl solution (100 ml) and dried with Na₂SO₄, filtered and evaporated. The residue was chomatographed over SiO₂ (Merck 230-400 mesh) eluting with EtOAc-nHexane (2:3), producing 5-hydroxy-indan-1-one (3.55g, 22.6 mmol, 45.9 %) as orange crystals Mp. 185-187 °C, MS m/e= 148.2 (M⁺).

### Example 60

### (RS)-1-(5-Methoxy-2,3-dihydro-1H-indol-2-ylmethyl)-4-(4-methyl-benzyl)-piperidin-4-ol.

To a solution of (RS)-1-[5-methoxy-1-(toluene-4-sulfonyl)-2,3-dihydro-1H-indol-2-ylmethyl]-4-(4-methyl-benzyl)-piperidin-4-ol (300 mg, 0.576 mmol) in toluene (15 ml) was added sodium bis(2-methoxy-ethoxy) aluminium hydride (3.5 M in THF) (0.66 ml, 2.31 mmol), and the mixture refluxed 18 hr. After cooling, 2N NaOH was added to pH 14, the mixture was extracted with EtOAc (3x 25ml) and the combined extracts washed with satd. NaCl (25 ml), dried with (Na₂SO₄) then filtered and evaporated. The residue was chromatographed over SiO₂ (Merck 230-400 mesh) eluting with EtOAc-cyclohexane-Et₃N (9:10:1) to afford (RS)-1-(5-methoxy-2,3-dihydro-1H-indol-2-ylmethyl)-4-(4-methyl-benzyl)-piperidin-4-ol as a viscous yellow oil (158.4 mg, 0.433 mmol, 75%), MS: m/e= 367.3 (M+H⁺).

### Example 61

### (RS)-1-[5-methoxy-1-(toluene-4-sulfonyl)-2,3-dihydro-1H-indol-2-ylmethyl]-4-(4-methyl-benzyl)-piperidin-4-ol.

(RS)-Toluene-4-sulfonic acid 5-methoxy-1-(toluene-4-sulfonyl)-2,3-dihydro-1H-indol-2-ylmethyl ester (200 mg, 0.41 mmol) and 4-(4-methyl-benzyl)-piperidin-4-ol (337 mg, 1.64 mmol) were heated in mesitylene (10 ml) for 20 hr at 140 °C. After cooling 4N HCl was added to pH1 and the mixture extracted with EtOAc (3x 25 ml), the extracts dried (Na₂SO₄), filtered and evaporated. Purification of the crude material over SiO₂ (Merck 230-400 mesh) eluting with EtOAc-cyclohexane-Et₃N (9:10:1) afforded the title compound as a yellow oil (155.9 mg, 0.3 mmol, 73 %), MS: m/e= 521.4 (M+H⁺).

### Example 62

### (RS)-Toluene-4-sulfonic acid 5-methoxy-1-(toluene-4-sulfonvl)-2,3-dihydro-1H-indol-2-ylmethyl ester

(RS)-(5-methoxy-2,3-dihydro-1H-indol-2-yl)-methanol (100 mg, 0.46 mmol), *p*-toluenesulfonyl chloride (177 mg, 0.93 mmol), triethylamine (0.21 ml, 1.48 mmol) and N,N-dimethylamino pyridine (2 mg) were stirred for 18 hr at RT in CH₂Cl₂ (5 ml). 1N HCl (10 ml) was added, the mixture extracted with CH₂Cl₂ (2x 25 ml) and the extracts washed with satd. NaCl solution (20 ml) and dried (Na₂SO₄). The crude material was chromatographed over SiO₂ (Merck 230-400 mesh) eluting with EtOAc-cyclohexane-Et₃N (9:10:1) to afford the title compound as a yellow oil (172.8 mg, 0.35 mmol, 76 %), MS: m/e= 488.0 (M+H⁺).

### Example 63

### (RS)-(5-Methoxy-2,3-dihydro-1H-indol-2-yl)-methanol

(RS)-5-methoxy-2,3-dihydro-1H-indole-2-carboxylic acid methyl ester (2.5 g, 12.06 mmol) in THF (130 ml) was added dropwise to a suspension of LiAlH₄ in THF (80 ml) at 4 °C, and the mixture allowed to stir at RT overnight, and then heated at 65 °C for 4 hr. After cooling, H20 (20 ml) was added cautiously and the mixture stirred 20 min, then filtered and the filtrate extracted with EtOAc (3x 50 ml), the extracts were washed with satd. NaCl solution (50 ml), dried (Na₂SO₄) filtered and evaporated. The crude oil was chromatographed over SiO₂ (Merck 230-400 mesh) eluting with CH₂Cl₂-MeOH (9:1) affording (RS)-(5-methoxy-2,3-dihydro-lH-indol-2-yl)-methanol as a yellow oil (1.41 g, 7.86 mmol, 65 %), MS: m/e= 179.1 (M⁺).

### Example 64

### (RS)-5-Methoxy-2,3-dihydro-1H-indole-2-carboxylic acid methyl ester

To 5-methoxy-1H-indole-2-carboxylic acid ethyl ester (5 g, 22.8 mmol) in MeOH (150 ml) was added magnesium turnings (2.2 g, 91.6 mmol, 4eq.) and the mixture vigorously mechanically stirred (10-30 °C) for 2 hr. 4N HCl was added (to pH1-2), then 25% NH₄OH (to pH 7), and the mixture extracted with EtOAc (4x 100 ml). The combined extracts were then washed with satd. NaCl solution (50 ml), dried (Na₂SO₄) filtered and evaporated. The crude green oil was chromatographed over SiO₂ (Merck 230-400 mesh) eluting with EtOAc-nHexane (1:3) to afford the title compound (3.21g, 15.5 mmol, 68 %) as a yellow solid Mp. 59-61 °C, MS: m/e= 207.1 (M⁺).

### Example 65

### 5-Methoxy-1H-indole-2-carboxylic acid ethyl ester

5-Methoxy-1H-indole-2-carboxylic acid (10 g, 52.3 mmol) in EtOH (400 ml) containing 36% H₂SO₄ (7 ml) was heated under reflux for 18 hr. After cooling the mixture was neutralised with 2N NaOH (to pH 7) and extracted with EtOAc (3x 150ml), the combined extracts were washed with 10% NaHCO₃ (2x 25 ml), dried (MgSO₄), filtered and evaporated to afford 5-methoxy-1H-indole-2-carboxylic acid ethyl ester as a brown solid (9.52 g, 43.3 mmol, 82 %) Mp. 154-155 °C, MS: m/e= 219.1 (M⁺).

### Preparation of the intermediates for examples 22 and 23

### Example 66

### (RS)-N-(2-Bromomethyl-2,3-dihydro-benzofuran-5-yl)-methansulfonamide

(RS)-2-bromomethyl-2,3-dihydro-benzofuran-5-ylamine (410 mg, 1.80 mmol), methansulfonyl-chloride (206 mg, 1.8 mmol), Et₃N (182 mg, 1.80 mmol) were dissolved in CH₂Cl₂ (50 ml) and stirred overnight at room temperature. Solvent was then evaporated and the residue was chromatographed over SiO₂ (Merck 230-400 mesh) eluting with CH₂Cl₂-MeOH (95:5) to provide (RS)-N-(2-bromomethyl-2,3-dihydro-benzofuran-5-yl)-methansulfonamide (516 mg, 94%) as an oil. MS: m/e= 305.9 (M-H⁺)

(RS)-2-bromomethyl-2,3-dihydro-benzofuran-5-ylamine was prepared following the procedure described in example 44.

### Preparation of the intermediates for examples 24 and 25.

### Example 67

### (RS)-1-(6-Methoxy-2,3-dihydro-benzofuran-2-ylmethyl)-4-(4-methyl-benzyl)-piperidin-4-ol

(RS)-toluene-4-sulfonic acid 6-methoxy-2,3-dihydro-benzofuran-2-ylmethyl ester (110 mg, 0.33 mmol) and 4-(4-methyl-benzyl)-piperidin-4-ol (148 mg, 0.72 mmol) were dissolved in DMF (3ml) and heated at 130 °C for 1 h. DMF was then evaporated, the residue was dissolved in CH₂Cl₂ (5 ml) and washed with H₂O (5 ml). Organic phase was dried over Na₂SO₄, and concentrated. The residue was chromatographed over SiO₂ (Merck 230-400 mesh) eluting with CH₂Cl₂-MeOH(19:1) to provide (RS)-1-(6-methoxy-2,3-dihydro-benzofuran-2-ylmethyl)-4-(4-methyl-benzyl)-piperidin-4-ol (80 mg, 66%) as a yellow oil, MS:m/e=368.4 (M+H⁺).

### Example 68

### (RS)-Toluene-4-sulfonic acid 6-methoxy-2,3-dihydro-benzofuran-2-ylmethyl ester

To a solution of (RS)-(6-methoxy-2,3-dihydro-benzofuran-2-yl)-methanol (0.09 g, 0.5 mmol), Et₃N (0.1 ml, 0.75 mmol), DMAP (1 mg, 0.01 mmol) in CH₂Cl₂ (1.5 ml) at 0°C was added dropwise a solution of *p*--toluenesulfonylchloride (115 mg, 0.6 mmol). The reaction mixture was allowed to warm to room temperature and was stirred during 4 hr before the addition of H₂O (2 ml). The aqueous phase was extracted with CH₂Cl₂ (2x5 ml). Combined organic phases were dried over Na₂SO₄, and concentrated. The residue was chromatographed over SiO₂ (Merck 230-400 mesh) eluting with nHexane-EtOAc (4:1) to provide (RS)-toluene-4-sulfonic acid 6-methoxy-2,3-dihydro-benzofuran-2-ylmethyl ester (120 mg, 72%) as a colorless oil, MS:m/e=334 (M⁺).

### Example 69

### 1-(6-Methoxy-benzofuran-2-ylmethyl)-4-(4-methyl-benzyl)-piperidin-4-ol

To a solution of 6-methoxy-2-benzofuranmethanol (89 mg, 0.5 mmol) in dioxan (3 ml) at room temperature was added dropwise SOCl₂ (0.11 ml, 1.5 mmol). After 1.5 hr, the reaction mixture was concentrated at room temperature under high vacuum. The residue was dissolved in dioxan (3 ml) and treated with 4-(4-methyl-benzyl)-piperidin-4-ol (225 mg, 1.1 mmol). After stirring 17 hr at room temperature, the solvent was evaporated. The residue was taken up in H₂O (4 ml) and extracted with CH₂Cl₂(6x4 ml). The combined organic phases were dried over Na₂SO₄, and concentrated. The residue was chromatographed over SiO₂ (Merck 230-400 mesh) eluting with CH₂Cl₂-MeOH (19:1) to provide 1-(6-methoxy-benzofuran-2-ylmethyl)-4-(4-methyl-benzyl)-piperidin-4-ol (140 mg, 77%) as a yellow oil, MS:m/e=366.3 (M+H⁺).

### Example 70

### (RS)-(6-Methoxy-2,3-dihydro-benzofuran-2-yl)-methanol

A solution of (RS)-6-methoxy-2,3-dihydro-benzofuran-2-carboxylic acid (158 mg, 0.813 mmol) in THF (2 ml) was added dropwise in a 0 °C suspension of LiAlH₄ (31 mg, 0.813 mmol) in THF (2 ml). After 30 min stirring at 0°C, the reaction mixture was allowed to reflux for 30 minutes. The reaction mixture was then cooled to 0°C and treated successively with H₂O (0.05 ml), 5N NaOH (0.05 ml), H₂O (0.15 ml). After 20 minutes stirring at room temperature, EtOAc was added followed by Na₂SO₄. The so obtained solid was filtered and the filtrate was evaporated. The residue was chromatographed over SiO₂ (Merck 230-400 mesh) eluting with nHexane-EtOAc (4:1) to provide (RS)-(6-methoxy-2,3-dihydro-benzofuran-2-yl)-methanol (102 mg, 70%) as a colorless oil, MS:m/e=180 (M⁺).

Following the method of example 70 the compound of example 71 was prepared.

### Example 71

### 6-Methoxy-2-benzofuranmethanol

The title compound MS: m/e=178 (M⁺) was prepared from 6-methoxy-2-benzofurancarboxylic acid.

Following the method of example 53 the compound of example 72 was prepared.

### Example 72

### (RS)-6-Methoxy-2.3-dihydro-benzofuran-2-carboxylic acid

The title compound Mp. 108-110 °C MS: m/e=194 (M⁺) was prepared from 6-methoxy-2-benzofurancarboxylic acid.

6-Methoxy-2-benzofurancarboxylic acid was prepared according to the literature:
S. Tanaka , *J*. *Am*. *Chem*. *Soc.* 73, **1951,** 872

### Example 73

### (RS)-4-Benzyl-1-(6-benzyloxy-chroman-2-ylmethyl)-piperidin-4-ol

(RS)-6-Benzyloxy-2-bromomethyl-chroman (0.52 g, 1.56 mmol) and 4-(benzyl)-piperidin-4-ol (0.66 g, 3.43 mmol) in toluene (20 ml) was refluxed for 18 hr under argon. After removal of the solvent the crude mixture was chromatographed over SiO₂ (Merck 230-400 mesh) CH₂Cl₂-MeOH-NH₄OH (100:5:0.25) to afford the title compound (RS)-4-benzyl-1-(6-benzyloxy-chroman-2-ylmethyl)-piperidin-4-ol as a yellow oil (0.6 g, 1.35 mmol, 86 %), MS: m/e= 444.5 (M+H⁺).

### Example 74

### (RS)-6-Benzyloxy-2-bromomethyl-chroman

(RS)-6-Benzyloxy-(chroman-2-yl)-methanol (0.97 g, 3.58 mmol) and 1,1'-carbonyldiimidazole (0.58 g, 3.58 mmol) and allyl bromide (19.9 mmol) were heated in acetonitrile at 80° C for 4 hr. The crude mixture was then evaporated to dryness and chomatographed over SiO₂ (Merck 230-400 mesh) eluting with CH₂Cl₂. This afforded (RS)-6-benzyloxy-2-bromomethyl-chroman (0.28 g, 0.84 mmol, 23 %) as a white solid Mp. 61-63 °C, MS m/e= 332.0 (M⁺).

### Example 75

### (RS)-6-Benzyloxy-(chroman-2-yl)-methanol

To a stirred suspension of LiAlH₄ (0.352 g, 9.26 mmol) in THF at 10 °C was added a solution of (RS)-6-hydroxy-chroman-2-carboxylic acid (1.2 g, 6.18 mmol) in THF (25 ml) over 15 min. After allowing to warm to ambient temperature, 4N HCl (25 ml) and EtOAc (100 ml) was added and the mixture shaken. The aqueous phase was extracted with EtOAc (100 ml) and the combined organic extracts were washed with satd. NaCI (50 ml), dried with Na₂SO₄ and evaporated. The resulting solid was taken up in acetone (50 ml) then K₂CO₃ (0.94 g, 6.8 mmol) and benzyl bromide (0.81 ml, 6.8 mmol) were added and the mixture refluxed for 18 hr. The reaction mixture was filtered, evaporated to dryness and chromatographed over SiO₂ (Merck 230-400 mesh) eluting with EtOAc-cyclohexane (3:7) to afford (RS)-6-benzyloxy-(chroman-2-yl)-methanol (1.0 g, 3.7 mmol, 60%) as a white solid Mp. 80-82 °C, MS m/e= 270.1 (M⁺).

### Example 76

### (RS)-6-Hydroxy-chroman-2-carboxylic acid

(RS)-6-Methoxy-chroman-2-carboxylic acid (1.12 g, 5.37 mmol) and pyridinium hydrochloride (11.2 g, 96.6 mmol) were heated together at 180 °C for 2 hr under argon with stirring. After cooling to ambient temperature H₂O (100 ml) and EtOAc (50 ml) was added and shaken. The aqueous phase was extracted with EtOAc (50 ml) and the combined organic extracts were washed with satd. NaCl solution (50 ml) and then dried with Na₂SO₄ and evaporated to afford (RS)-6-hydroxy-chroman-2-carboxylic acid as white crystals (0.94 g, 4.84 mmol, 90 %) Mp. 175-177 °C, MS m/e= 194.1 (M⁺).

### Example 77

### (RS)-6-Methoxy-chroman-2-carboxylic acid

A vigorously stirred mixture of 6-methoxychromenone-2-carboxylic acid (2.20 g, 10 mmol) and Pd/C (10%), (300 mg) in acetic acid (200 ml) was heated at 60 °C for 5 hr under an atmosphere of hydrogen. The catalyst was removed and the solution evaporated to afford (RS)-6-methoxy-chroman-2-carboxylic acid (2.04 g, 9.8 mmol, 98%) as yellowish crystals Mp. 131-135 °C, MS m/e= 208.1 (M⁺). Lit: N. Cohen et. al. *J*. *Med Chem.,* **1989,** 32, 1842-1860.; and D.T. Witaik et. al, *J*. *Med*. *Chem.,* **1971,** 14, 758-766.

### Example 78

### (1RS,2RS)-1-(6-Benzyloxy-1-hydroxy-1,2,3,4-tetrahvdro-naphthalen-2-ylmethvl)-4-(4-methyl-benzyl)-piperidin-4-ol

(RS)-6-Benzyloxy-2-[4-hydroxy-4-(4-methyl-benzyl)-piperidin-1-ylmethyl]-3,4-dihydro-2H-naphthalen-1-one (1.30 g, 2.77 mmol) in THF (15 ml) was added dropwise to a suspension of LiAlH₄ (0.6 g, 15.5 mmol) in THF (20 ml) over 15 min (5-10 °C) and then stirred at RT for 2.5 hr. Distilled H₂O (1 ml) and 4N NaOH (2 ml) followed by further H₂O (2 ml), was added to quench the reaction and stirred vigorously 15 min., the mixture was then dried with Na₂SO₄, filtered and evaporated. The resulting crude liquid was chromatographed over SiO₂ (Merck 230-400 mesh) eluting with CH₂Cl₂-MeOH-NH₄OH (100:5:0.25) to afford the title compound as a white foam (0.75 g, 1.6 mmol, 57 %), MS m/e= 472.4 (M+H⁺).

Following the general method of example 78, example 79 was prepared.

### Example 79

### (1RS,2RS)-1-(6-Benzyloxy-1-hydroxy-1,2,3,4-tetrahvdro-naphthalen-2-ylmethyl)-4-benzyl-piperidin-4-ol

The title compound was obtained as a colourless oil MS: m/e=458.6 (M+H⁺), prepared from (RS)-2-(4-benzyl-4-hydroxy-piperidin-1-ylmethyl)-6-benzyloxy-3,4-dihydro-2H-naphthal-en-1-one.

### Example 80

### (RS)-6-Benzyloxy-2-[4-hydroxy-4-(4-methyl-benzyl)-piperidin-1-ylmethyl]-3,4-dihydro-2H-naphthalen-1-one

6-Benzyloxy-1-tetralone (1.26 g, 5 mmol), 4-(4-methyl-benzyl)-piperidin-4-ol hydrochloride (1.20 g, 5 mmol) and paraformaldehyde (150 mg, 5 mmol) in DMF (10 ml) were heated at 70 °C for 17 hr. The crude mixture was partitioned between distilled H₂O (150 ml), 25 % NH₄OH (2 ml) and EtOAc (100 ml). The aqueous phase was further extracted with EtOAc (100 ml) and the combined organic extracts washed twice with satd NaCl solution (100 ml), dried Na₂SO₄ filtered and evaporated to afford an orange oil. Chromatography over SiO₂ (Merck 230-400 mesh) eluting with EtOAc-Et₃N (19:1) afforded (RS)-6-benzyloxy-2-[4-hydroxy-4-(4-methyl-benzyl)-piperidin-1-ylmethyl]-3,4-dihydro-2H-naphthalen-1-one as an amber oil (1.34 g, 2.85 ml, 57 %), MS m/e= 470.3 (M+H⁺).

Following the general method of example 80, example 81 was prepared

### Example 81

### (RS)-2-(4-Benzyl-4-hydroxy-piperidin-1-ylmethyl)-6-benzyloxy-3,4-dihydro-2H-naphtalen-1-one.

The title compound was obtained as a yellow oil MS: m/e=456.6 (M+H⁺), prepared from 6-benzyloxy-1-tetralone, 4-benzyl-4-hydroxy-piperidine hydrochloride and paraformaldehyde.

### Example 82

### 6-Benzyloxy-1-tetralone

6-Hydroxy-1-tetralone (10 g, 61.65 mmol), benzyl bromide (8.1 ml, 67.82 mmol) and K₂CO₃ (21.3 g, 154 mmol) in acetone (40 ml) were heated under reflux 2.5 hr. Following filtration and evaporation *of* the solvent the crude material was acidified to pH1 with 1N HCl and partioned between H₂O (150 ml) and EtOAc (150 ml). The aqueous phase was further extracted with EtOAc (100 ml) and the extracts dried (Na₂SO₄) filtered and evaporated to afford 6-benzyloxy-1-tetralone as an orange solid (12.37 g, 49.3 mmol, 80 %) Mp. 97-100 °C, MS m/e= 252.1 (M⁺).

### Example 83

### 6-Hydroxy-1-tetralone

6-Methoxy-1-tetralone (80 g, 453 mmol) was taken up in 48% HBr (270 ml) at 4 °C, then stirred at ambient temperature for 4 hr. Distilled H₂O (11) was added with cooling to 4 °C and the precipitated solid was filtered, washed with H20 and recrystallised twice from EtOH-H₂O (4:1) to afford 6-hydroxy-1-tetralone as a beige solid (59.7g, 368 mmol, 81 %), Mp. 153-155 °C, MS m/e= 162.1 (M⁺).

### Example 84

### (RS)-1-(6-Methoxy-1,2,3,4-tetrahydro-naphthalen-2-ylmethyl)-4-(4-methyl-benzyl)-piperidin-4-ol

(RS)-Toluene-4-sulfonic acid 6-methoxy-1,2,3,4-tetrahydro-naphthalen-2-ylmethyl ester (1.32 g, 3.81 mmol) and 4-(methyl-benzyl)-piperidin-4-ol (3.13 g, 15.24 mmol) in mesitylene (100 ml) was heated at 140 °C for 20 hr. Following evaporation of the solvent the crude material was partioned between CH₂Cl₂ (50 ml) and 5% NaHCO₃ (30 ml), the aqueous phase was extracted with CH₂Cl₂ (2x100 ml) washed with satd. NaCl solution (50 ml), dried (Na₂SO₄), filtered and evaporated. The crude product was chomatographed over SiO₂ (Merck 230-400 mesh) eluting with EtOAc-nHexane (1:3) then (1:1), to afford (RS)-1-(6-methoxy-1,2,3,4-tetrahydro-naphthalen-2-ylmethyl)-4-(4-methyl-benzyl)-piperidin-4-ol as a white solid (972 mg, 2.56 mmol, 67.3 %), Mp. 91-94 °C, MS m/e= 380.3 (M+H⁺).

### Example 85

### (RS)-Toluene-4-sulfonic acid 6-methoxy-1,2,3,4-tetrahydro-naphthalen-2-ylmethyl ester

(RS)-(6-methoxy-1,2,3,4-tetrahydro-naphthalen-2-yl)-methanol (939 mg, 4.88 mmol), *p*-toluenesulfonyl chloride (934 mg, 4.9 mmol), triethylamine (1.12 ml, 7.88 mmol) and N,N-dimethyl aminopyridine (10 mg) in CH₂Cl₂ (10 ml) was stirred together at ambient temperature for 24 hr. Acidification with 1N HCl (pH 1-2), extraction of the aqueous phase with CH₂Cl₂ (2x 20 ml), drying (Na₂SO₄) filtration and evaporation afforded the crude product which was chomatographed over SiO₂ (Merck 230-400 mesh) eluting with EtOAc-nHexane (1:3) to afford the title compound as a colourless oil (1.37 g, 3.95 mmol, 81 %), MS m/e= 346.1 (M⁺).

### Example 86

### (RS)-(6-Methoxy-1,2,3,4-tetrahydro-naphthalen-2-yl)-methanol

(RS)-6-methoxy-1,2,3,4-tetrahydro-naphthalene-2-carboxylic acid ethyl ester (1.39 g, 5.93 mmol) in THF (20 ml) was added dropwise to a suspension of LiAlH₄ (473 mg, 12.46 mmol) at 0-10 °C over 15 min. The reaction was stirred for 1 hr at RT, then quenched by the addition of 4N NaOH (15 ml) and water (20 ml). After stirring vigorously for 15 min the mixture was extracted with EtOAc (25 ml), the aqueous phase was further extracted with EtOAc (2x 25 ml), the combined organic extracts were washed with satd. NaCl solution (30 ml), dried (Na₂SO₄) filtered and evaporated to give the crude product as a clear oil. Chromatographic purification over SiO₂ (Merck 230-400 mesh) eluting with EtOAc-nHexane (1:3) produced (RS)-(6-methoxy-1,2,3,4-tetrahydro-naphthalen-2-yl)-methanol as a colourless oil (989 mg, 5.14 mmol, 86 %), MS m/e= 192.1 (M⁺).

### Example 87

### (RS)-6-Methoxy-1,2,3,4-tetrahvdro-naphthalene-2-carboxylic acid ethyl ester

(RS)-6-methoxy-1-oxo-1,2,3,4-tetrahydro-naphthalene-2-carboxylic acid ethyl ester (2.0 g, 8.05 mmol) in MeOH (20 ml) and Pd/C (10%), (200 mg) was stirred vigorously under a hydrogen atmosphere for 3 hr at ambient temperature. The catalyst was removed and solvent evaporated to afford an oil, which was purified by chromatography over SiO₂ (Merck 230-400 mesh) eluting with EtOAc-nHexane (1:9) to afford (RS)-6-methoxy-1,2,3,4-tetrahydro-naphthalene-2-carboxylic acid ethyl ester as a colourless oil (1.88 g, 6.14 mmol, 76 %), MS m/e= 234.1 (M⁺).

### Example 88

### (RS)-6-Methoxy-1-oxo-1,2,3,4-tetrahydro-naphthalene-2-carboxylic acid ethyl ester

6-Methoxy-tetralone (10 g, 56.75 mmol), diethylcarbonate (20.6 ml, 170.2 mmol) and sodium hydride (5.06 g, 210.8 mmol) were heated together at 65 °C in THF (400 ml) for 18 hr. After cooling glacial acetic acid (15 ml, 250 mmol) was added cautiously followed by toluene (2x200 ml) then co-evaporated to remove excess acetic acid. The residue was taken up in EtOAc (400 ml), H₂O (400 ml) and shaken, the aqueous phase was extracted with EtOAc (2x100 ml) and the combined organic extracts washed with satd. NaCl solution (100 ml), dried (Na₂SO₄), filtered and washed. The crude product was crystallised from EtOAc-nHexane to afford the title compound as a yellow solid (8.3 g, 33.4 mmol, 58 %), MS m/e= 248.1 (M⁺).

## Claims

1. A compound of the formula 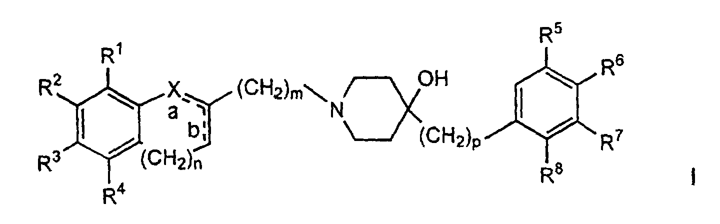 wherein
X denotes -O-, -NH-, -CH₂-, -CH=, -CHOH-, -S-, -SO- or -SO₂-;
R¹-R⁴ are, independently from each other hydrogen, hydroxy, C₁-C₄-alkyl-sulfonylamino, 1- or 2-imidazolyl or acetamido;
R⁵-R⁸ are, independently from each other hydrogen, hydroxy, C₁-C₄-alkyl, halogen, C₁-C₄-alkoxy, trifluoromethyl or trifluoromethyloxy;
a and b are, independently from each other, double bonds or not, wit the proviso that when "a" is a double bond, "b" cannot be a double bond;
n is 0-2;
m is 1-3;
p is 1
and pharmaceutically acceptable addition salts thereof.

2. A compound according to claim 1, wherein X is -O-.

3. A compound according to claim 2, selected from the following group:
(RS)-1-(5-hydroxy-2,3-dihydro-benzofuran-2-ylmethyl)-4-(4-methyl-benzyl)-piperidine-4-ol,
(RS)-4-benzyl-1-(5-hydroxy-2,3-dihydro-benzofuran-2-ylmethyl)-piperidine-4-ol,
(RS)-4-(4-fluoro-benzyl)-1-(5-hydroxy-2,3-dihydro-benzofuran-2-ylmethyl)-piperidine-4-ol,
(RS)-4-(4-ethyl-benzyl)-1-(5-hydroxy-2,3-dihydro-benzofuran-2-ylmethyl)-piperidine-4-ol,
(S)-1-(5-hydroxy-2,3-dihydro-benzofuran-2-ylmethyl)-4-(4-methyl-benzyl)-piperidine-4-ol,
(S-1-(5-hydroxy-2,3-dihydro-benzofuran-2-ylmethyl)-4-(4-chloro-benzyl)-piperidine-4-ol,
(RS)-N-[2-{4-hydroxy-4-(4-methyl-benzyl)-piperidine-1-ylmethyl}-2,3-dihydrobenzofuran-5-yl]-methane and dihydrobenzofuran-5-yl]-methane sulfonamide and
(RS)-N-[2-{4-hydroxy-4-(4-methyl-benzyl)-piperidine-1-ylmethyl}-2,3-dihydrobenzofuran-5-yl]-methane sulfonamide.

4. A compound according to claim 1, wherein X is -CHOH-.

5. A compound according to claim 4, selected from the following group:
(1RS,2RS) and (1RS,2SR)-2-[4-hydroxy-4-(4-methyl-benzyl)-piperidine-1-ylmethyl]-indan-1,5-diol,
(1RS,2RS)-1-(1,6-dihydroxy-1,2,3,4-tetrahydro-naphthalen-2-ylmethyl)-4-(4-methyl-benzyl)-piperidine-4-ol and
(1RS,2RS)-2-(4-benzyl-4-hydroxy-piperidine-1-yl-methyl)-6-hydroxy-1,2,3,4-tetrahydronaphthalen-1-ol.

6. A compound according to claim 1, wherein X is -CH2- or -CH=.

7. A compound according to claim 6, selected from the following group:
(RS)-1-(5-hydroxy-indan-2-ylmethyl)-4-(4-methyl-benzyl)-piperidine-4-ol and
(RS)-1-(6-hydroxy-1,2,3,4-tetrahydro-naphthalen-2-ylmethyl)-4-(4-methyl-benzyl)-piperidine-4-ol.

8. A compound according to claim 1, wherein X is -NH-.

9. A compound according to claim 8, selected from the following group:
(RS)-2- [4-hydroxy-4-(4-methyl-benzyl)-piperidine-1-ylmethyl]-2,3-dihydro- lH-indol-5-ol.

10. A compound according to claim 1, wherein X is -S-, -SO- or -SO₂-.

11. A medicament containing one or more compounds of any one of claims 1 to 10 or a pharmaceutically acceptable salt thereof for the treatment of diseases.

12. A medicament according to claim 11 for the treatment of diseases based on therapeutic indications for NMDA receptor subtype specific blockers, which include acute forms of neurodegeneration caused, e.g., by stroke and brain trauma, and chronic forms of neurodegeneration such as Alzheimer's disease, Parkinsons's disease, Huntington's disease, ALS (amyotrophic lateral sclerosis) and neurodegeneration associated with bacterial sclerosis) and neurodegeneration associated with bacterial or viral infections.

13. A process for preparing a compound of formula I as defined in claim 1, which process comprises:
a) reacting a compound of the formula 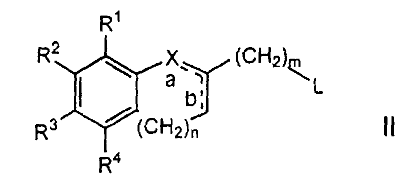
wherein R¹-R⁴, X, a, b, n and m have the meaning given in claim 1 and L is OH or a leaving group, for example, halogen or -O-tosyl,
with a compound of the formula 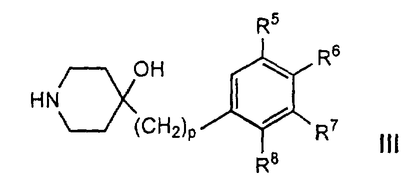
wherein R⁵-R⁸ and p have the significances given in claim 1,
or
b) dehydrating a compound of the formula 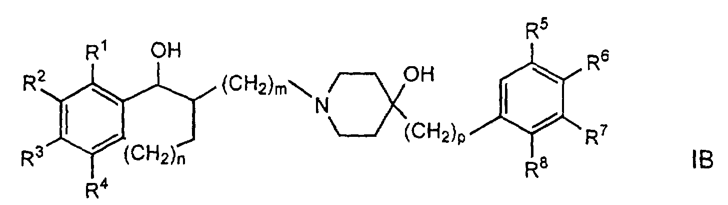 to give a compound of the formula 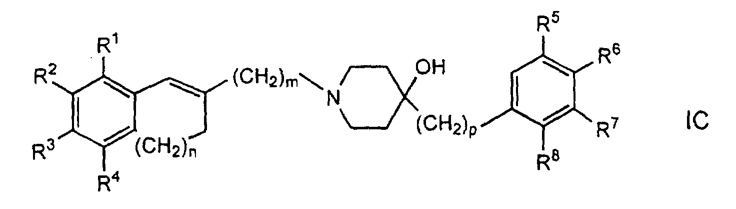 wherein m is 1 and the other substituents have the significances given in claim 1, or
c) reducing a compound of the formula 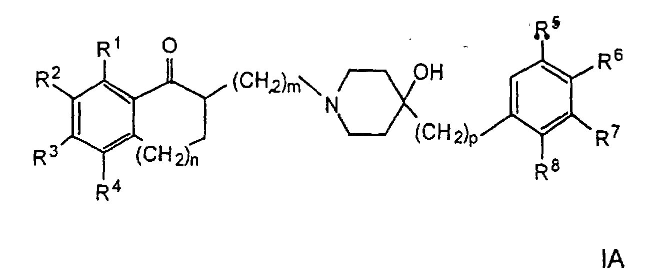 to give a compound of the formula IB, or
d) debenzylating a compound of the formula 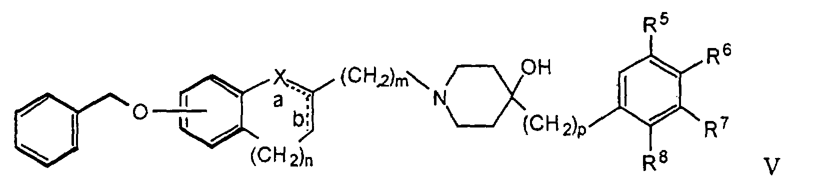 wherein the substituents have the significances given in claim 1,
or
e) reacting a compound of formula I, wherein one of R¹-R⁴ is an amino group with a C₁-C₄-alkyl-sulfonyl halogen to give a compound of formula I, wherein one of R¹-R⁴ is a C₁-C₄-alkyl-sulfonyl-amino group, or
f) hydrogenating the isolated double bond in a compound of formula I, or
g) cleaving off (a) hydroxy or amino protecting group(s) present as (a) substituent(s) R¹-R⁴ or as X' = -N(protecting group)-, or
h) oxidizing a compound of formula I, wherein X represents -S- or -SO- to yield the corresponding sulfonyl (-SO₂) compound, and
i) if desired, converting the compound of formula I obtained into a pharmaceutically acceptable addition salt.

14. Compounds according to any one of claims 1-10 whenever prepared by a process as claimed in claim 13 or by an equivalent method.

15. The use of a compound in accordance with any one of claims 1-10 in the manufacture of a medicament for the treatment of diseases based on therapeutic indications of NMDA receptor subtype specific blockers, which include acute forms of neurodegeneration caused e.g., by stroke and brain trauma, and chronic forms of neurodegeneration such as Alzheimer's disease, Parkinson's disease, Huntington's disease, ALS (amyotrophic laterial sclerosis) and neurodegeneration associated with bacterial or viral infections

## Patentansprüche

1. Verbindung der Formel 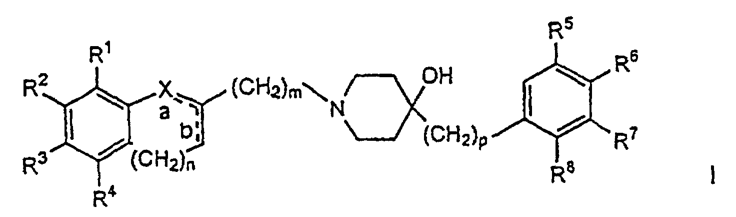
worin X -O-, -NH-, -CH₂-, -CH=, -CHOH-, -S-, -SO-oder -SO₂- bedeutet;
R¹-R⁴ unabhängig voneinander Wasserstoff, Hydroxy, C₁-C₄-Alkylsulfonylamino, 1- oder 2-Imidazolyl oder Acetamido darstellen;
R⁵-R⁸ unabhängig voneinander Wasserstoff, Hydroxy, C₁-C₄-Alkyl, Halogen, C₁-C₄-Alkoxy, Trifluormethyl oder Trifluor-methyloxy darstellen,
a und b unabhängig voneinander Doppelbindungen oder keine darstellen, mit der Maßgabe, dass, wenn "a" eine Doppelbindung ist, "b" keine Doppelbindung sein kann;
n 0-2 ist;
m 1 bis 3 ist;
p 1 ist
und pharmazeutisch verträgliche Additionssalze davon.

2. Verbindung nach Anspruch 1, worin X -O- darstellt.

3. Verbindung nach Anspruch 2, ausgewählt aus der nachstehenden Gruppe:
(RS)-1-(5-Hydroxy-2,3-dihydro-benzofuran-2-ylmethyl)-4-(4-methyl-benzyl)-piperidin-4-ol,
(RS)-4-Benzyl-1-(5-hydroxy-2,3-dihydro-benzofuran-2-ylmethyl)-piperidin-4-ol,
(RS)-4-(4-Fluor-benzyl)-1-(5-hydroxy-2,3-dihydro-benzofuran-2-ylmethyl)-piperidin-4-ol,
(RS)-4-(4-Ethyl-benzyl)-1-(5-hydroxy-2,3-dihydro-benzofuran-2-ylmethyl)-piperidin-4-ol,
(S)-1-(5-Hydroxy-2,3-dihydro-benzofuran-2-ylmethyl)-4-(4-methyl-benzyl)-piperidin-4-ol,
(S)-1-(5-Hydroxy-2,3-dihydro-benzofuran-2-ylmethyl)-4-(4-chlor-benzyl)-piperidin-4-ol,
(RS)-N-[2-{4-Hydroxy-4-(4-methyl-benzyl)-piperidin-1-ylmethyl}-2,3-dihydrobenzofuran-5-yl]-methansulfonamid und
(RS)-N-[2-{4-Hydroxy-4-(4-methyl-benzyl)-piperidin-1-ylmethyl}-2,3-dihydrobenzofuran-5-yl]-methansulfonamid.

4. Verbindung nach Anspruch 1, worin X -CHOH- darstellt.

5. Verbindung nach Anspruch 4, ausgewählt aus der nachstehenden Gruppe:
(1RS, 2RS) und (1RS, 2SR)-2-[4-Hydroxy-4-(4-methyl-benzyl)-piperidin-1-ylmethyl]-indan-1,5-diol,
(1RS,2RS)-1-(1,6-Dihydroxy-1,2,3,4-tetrahydro-naphth-2-ylmethyl)-4-(4-methyl-benzyl)-piperidin-4-ol und
(1RS,2RS)-2-(4-Benzyl-4-hydroxy-piperidin-1-yl-methyl)-6-hydroxy-1,2,3,4-tetrahydronaphth-1-ol.

6. Verbindung nach Anspruch 1, worin X -CH₂- oder -CH= darstellt.

7. Verbindung nach Anspruch 6, ausgewählt aus der nachstehenden Gruppe:
(RS)-1-(5-Hydroxy-indan-2-ylmethyl-4-(4-methyl-benzyl)-piperidin-4-ol und (RS)-1-(6-Hydroxy-1,2,3,4-tetrahydronaphth-2-ylmethyl)-4-(4-methyl-benzyl)-piperidin-4-ol.

8. Verbindung nach Anspruch 1, worin X -NH- darstellt.

9. Verbindung nach Anspruch 8, ausgewählt aus der nachstehenden Gruppe:
(RS)-2-[4-Hydroxy-4-(4-methyl-benzyl)-piperidin-1-yl-methyl]-2,3-dihydro-1H-indol-5-ol.

10. Verbindung nach Anspruch 1, worin X -S-, -SO-oder -SO₂- darstellt.

11. Arzneimittel, enthaltend eine oder mehrere Verbindungen nach einem der Ansprüche 1 bis 10 oder ein pharmazeutisch verträgliches Salz davon, zur Behandlung von Erkrankungen.

12. Arzneimittel nach Anspruch 11 zur Behandlung von Erkrankungen, die auf therapeutischen Indikationen von NMDA-Rezeptor-Subtyp-spezifischen Blockern basieren, die akute Formen von Neurodegeneration, verursacht beispielsweise durch Schlaganfall und Hirntrauma, und chronische Formen von Neurodegeneration, wie Alzheimer Krankheit, Parkinson'sche Krankheit, Huntington'sche Krankheit, ALS (amyotrophe laterale Sklerose), und Neurodegeneration, verbunden mit bakterieller Sklerose, und Neurodegeneration, verbunden mit bakteriellen oder viralen Infektionen, einschließen.

13. Verfahren zur Herstellung einer Verbindung der Formel I, wie in Anspruch 1 definiert, wobei das Verfahren umfasst:
a) Umsetzen einer Verbindung der Formel 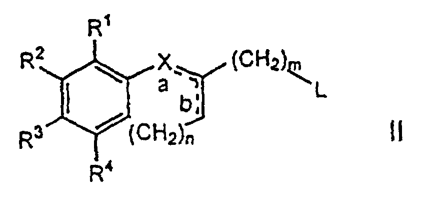
worin R¹-R⁴, X, a, b, n und m die in Anspruch 1 angegebene Bedeutung aufweisen und L OH oder eine Abgangsgruppe, beispielsweise Halogen oder -O-Tosyl, darstellt,
mit einer Verbindung der Formel 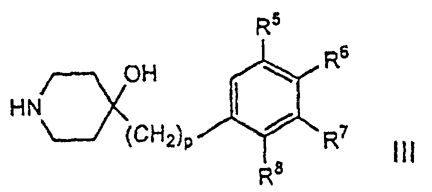
worin R⁵-R⁸ und p die in Anspruch 1 gegebenen Bedeutungen aufweisen, oder
b) Dehydratisieren einer Verbindung der Formel 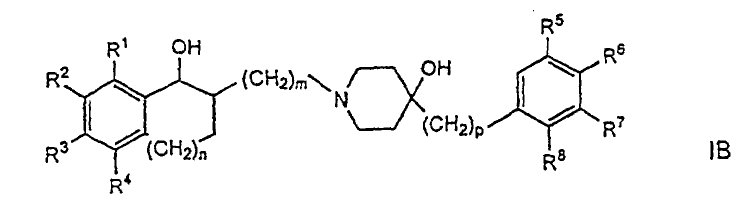
unter Gewinnung einer Verbindung der Formel 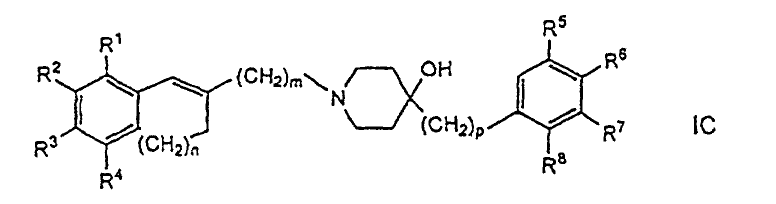
worin m 1 ist und die anderen Substituenten die in Anspruch 1 angegebenen Bedeutungen aufweisen, oder
c) Reduktion einer Verbindung der Formel 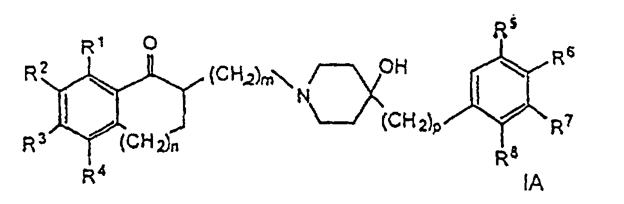 unter Gewinnung einer Verbindung der Formel IB, oder
d) Debenzylieren einer Verbindung der Formel 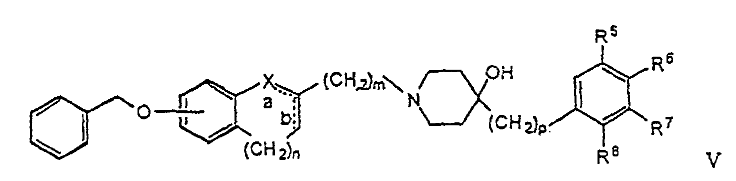 worin die Substituenten die in Anspruch 1 angegebenen Bedeutungen aufweisen, oder
e) Umsetzen einer Verbindung der Formel I, worin einer der Reste R¹-R⁴ eine Aminogruppe darstellt, mit einem C₁-C₄-Alkyl-sulfonylhalogen zu einer Verbindung der Formel I, worin einer der Reste R¹-R⁴ eine C₁-C₄-Alkylsulfonylaminogruppe darstellt, oder
f) Hydrieren der isolierten Doppelbindung in einer Verbindung der Formel I, oder
g) Abspalten von (einer) Hydroxy- oder Aminoschutzgruppe(n), die als (ein) Substituent(en) R¹-R⁴ oder als X' = -N(Schutzgruppe)- vorliegen, oder
h) Oxidieren einer Verbindung der Formel I, worin X -S- oder -SO- wiedergibt, unter Gewinnung der entsprechenden Sulfonyl(-SO₂)verbindung, und
i) falls erwünscht, Umwandeln der erhaltenen Verbindung der Formel I in ein pharmazeutisch verträgliches Additionssalz.

14. Verbindungen nach einem der Ansprüche 1 bis 10, hergestellt durch ein Verfahren nach Anspruch 13 oder durch ein äquivalentes Verfahren.

15. Verwendung einer Verbindung nach einem der Ansprüche 1 bis 10 bei der Herstellung eines Arzneimittels zur Behandlung von Erkrankungen, die auf therapeutischen Indikationen von spezifischen NMDA-Rezeptor-Subtyp-Blockern basieren, die akute Formen von Neurodegeneration, verursacht beispielsweise durch Schlaganfall und Gehirntrauma, und chronische Formen von Neurodegeneration, wie Alzheimer Krankheit, Parkinson'sche Krankheit, Huntington'sche Krankheit, ALS (amyotrophe laterale Sklerose) und Neurodegeneration, verbunden mit bakteriellen oder viralen Infektionen, einschließen.

## Revendications

1. Composé de formule 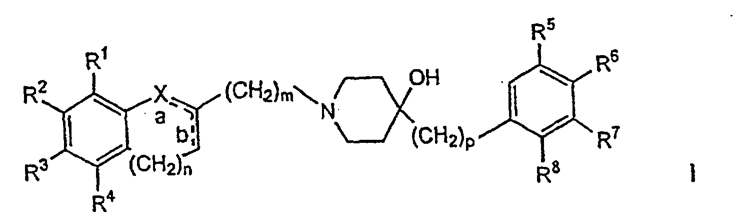 dans laquelle
x désigne -O-, -NH-, -CH₂-, -CH=, -CHOH-, -S-, -SO- ou -SO₂-;
R¹-R⁴ sont, indépendamment l'un de l'autre, un atome d'hydrogène ou un groupe hydroxy, alkyl(en C₁-C₄)sulfonylamino, 1- ou 2-imidazolyle ou acétamido;
R⁵-R⁸ sont, indépendamment l'un de l'autre, un atome d'hydrogène ou un groupe hydroxy, alkyle en C₁-C₄, halogéno, alcoxy en C₁-C₄, trifluorométhyle ou trifluorométhyloxy;
a et b indépendamment l'un de l'autre, sont ou non des doubles liaisons, à condition que, lorsque "a" est une double liaison, "b" ne puisse pas être une double liaison;
n vaut 0-2;
m vaut 1-3;
P vaut 1;
et sels pharmaceutiquement acceptables de ce composé.

2. Composé selon la revendication 1, dans lequel X est -O-.

3. Composé selon la revendication 2, choisi dans le groupe suivant:
le (RS)-1-(5-hydroxy-2,3-dihydrobenzofuran-2-ylméthyl)-4-(4-méthyl-benzyl)pipéridine-4-ol,
le (RS)-4-benzyl-1-(5-hydroxy-2,3-dihydrobenzofuran-2-ylméthyl)-pipéridine-4-ol,
le (RS)-4-(4-fluorobenzyl)-1-(5-hydroxy-2,3-dihydrobenzofuran-2-yl-méthyl)pipéridine-4-ol,
le (RS)-4-(4-éthylbenzyl)-1-(5-hydroxy-2,3-dihydrobenzofuran-2-yl-méthyl)pipéridine-4-ol,
le (S)-1-(5-hydroxy-2,3-dihydrobenzofuran-2-ylméthyl)-4-(4-méthyl-benzyl)pipéridine-4-ol,
le (S)-1-(5-hydroxy-2,3-dihydrobenzofuran-2-ylméthyl)-4-(4-chloro-benzyl)pipéridine-4-ol,
le (RS)-N-[2-{4-hydroxy-4-(4-méthylbenzyl)pipéridine-1-ylméthyl}-2,3-dihydrobenzofuran-5-yl]méthanesulfonamide et
le (RS)-N-[2-{4-hydroxy-4-(4-méthylbenzyl)pipéridine-1-ylméthyl}-2,3-dihydrobenzofuran-5-yl]méthanesulfonamide.

4. Composé selon la revendication 1, dans lequel X est -CHOH-.

5. Composé selon la revendication 4, choisi dans le groupe suivant:
le (1RS,2RS) et le (1RS,2SR)-2-[4-hydroxy-4-(4-méthylbenzyl)pipéridine-1-ylméthyl]indan-1,5-diol,
le (1RS,2RS)-1-(1,6-dihydroxy-1,2,3,4-tétrahydronaphtalén-2-ylméthyl)-4-(4-méthylbenzyl)pipéridine-4-ol et
le (1RS,2RS)-2-(4-benzyl-4-hydroxypipéridine-1-yl-méthyl)-6-hydroxy-1,2,3,4-tétrahydronaphtalén-1-ol.

6. Composé selon la revendication 1, dans lequel X est -CH₂- ou -CH=.

7. Composé selon la revendication 6, choisi dans le groupe suivant:
le (RS)-1-(5-hydroxyindan-2-ylméthyl)-4-(4-méthylbenzyl)pipéridine-4-ol et
le (RS)-1-(6-hydroxy-1,2,3,4-tétrahydronaphtalén-2-ylméthyl)-4-(4-méthyl-benzyl)pipéridine-4-ol.

8. Composé selon la revendication 1, dans lequel X est -NH-.

9. Composé selon la revendication 8, choisi dans le groupe suivant:
le (RS)-2-[4-hydroxy-4-(4-méthylbenzyl)pipéridine-1-ylméthyl]-2,3-dihydro-1H-indol-5-ol.

10. Composé selon la revendication 1, dans lequel X est -S-, -SO-ou -SO₂-.

11. Médicament contenant un ou plusieurs composés selon l'une quelconque des revendications 1 à 10 ou un de leurs sels pharmaceutiquement acceptables pour le traitement de maladies.

12. Médicament selon la revendication 11 pour le traitement de maladies à base d'indications thérapeutiques pour les agents bloquants spécifiques d'un sous-type de récepteur de NMDA, ce qui comprend des formes aiguës de neurodégénérescence provoquées, par exemple, par une attaque cérébrale et un traumatisme crânien, et des formes chroniques de neurodégénérescence telles que la maladie d'Alzheimer, la maladie de Parkinson, la maladie d'Huntington, l'ALS (sclérose latérale amyotrophique) et la neurodégénérescence associée à la sclérose bactérienne, et la neurodégé'
nérescence associée à des infections bactériennes ou virales.

13. Procédé de préparation d'un composé de formule I telle que définie dans la revendication 1, procédé qui comprend les étapes consistant à:
a) faire réagir un composé de formule 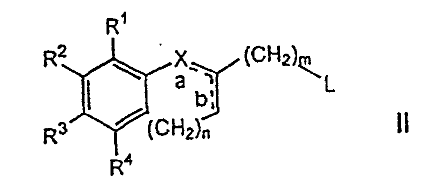
dans laquelle R¹-R⁴, X, a, b, n et m ont les significations données dans la revendication 1 et L est OH ou un groupe partant, for exemple halogéno ou -O-tosyle,
avec un composé de formule 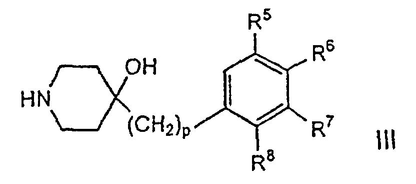
dans laquelle R⁵-R⁸ et p ont les significations données dans la revendication 1,
ou
b) déshydrater un composé de formule 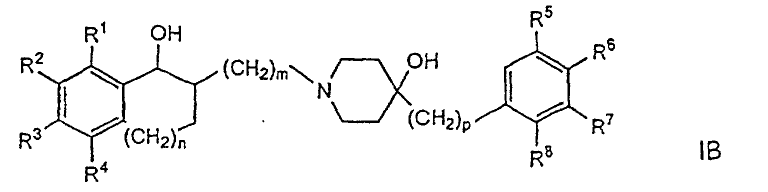 pour obtenir le composé de formule 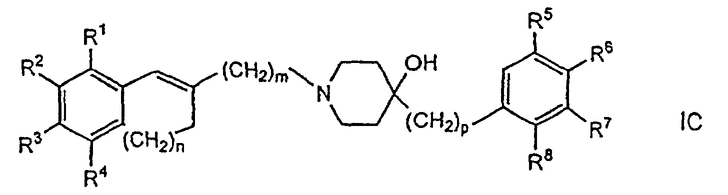 dans laquelle m vaut 1 et les autres substituants ont les significations données dans la revendication 1,
ou
c) réduire un composé de formule 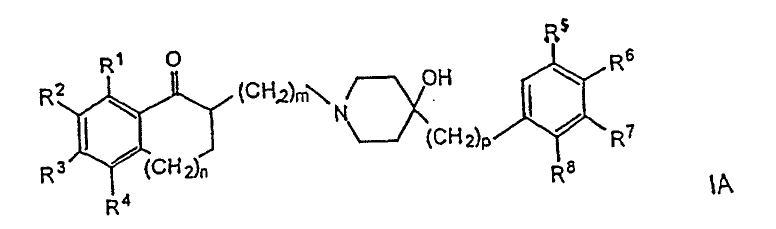 pour obtenir un composé de formule IB, ou
d) débenzyler un composé de formule 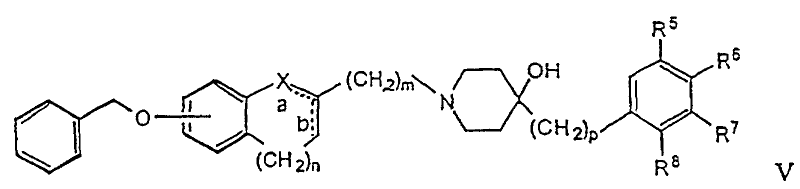 dans laquelle les substituants ont les significations données dans la revendication 1, ou
e) faire réagir un composé de formule I, dans laquelle un des radicaux R¹-R⁴ est un groupe amino, avec un halogénure d'alkylsulfonyle en C₁-C₄ pour obtenir un composé de formule I, dans laquelle un des radicaux R¹-R⁴ est un groupe alkyl(en C₁-C₄)sulfonylamino, ou
f) hydrogéner la double liaison isolée dans un composé de formule I, ou
g) éliminer (a) le(s) groupe(s) protecteur(s) de fonction hydroxy ou amino présent(s) en tant que (a) substituant(s) R¹-R⁴ ou que X' = -N(groupe protecteur)-, ou
h) oxyder un composé de formule I, dans laquelle X représente -S- ou -SO- pour obtenir le composé sulfonyle (-SO₂) correspondant, et
i) le cas échéant, convertir le composé de formule I obtenu en un sel d'addition d'acide pharmaceutiquement acceptable.

14. Composés selon l'une quelconque des revendications 1-10, préparés par un procédé selon la revendication 13 ou par un procédé équivalent.

15. Utilisation d'un composé selon l'une quelconque des revendications 1-10 dans la fabrication d'un médicament pour le traitement de maladies à base d'indications thérapeutiques pour les agents bloquants spécifiques d'un sous-type de récepteur de NMDA, ce qui comprend des formes aiguës de neurodégénérescence provoquées, par exemple, par une attaque cérébrale et un traumatisme crânien, et des formes chroniques de neurodégénérescence telles que la maladie d'Alzheimer, la maladie de Parkinson, la maladie d'Huntington, l'ALS (sclérose latérale amyotrophique) et la neurodégénérescence associée à des infections bactériennes ou virales.
